# EUROPEAN PATENT APPLICATION

(11) **EP 4 006 145 A1**
(43) Date of publication of application: **01.06.2022**
(21) Application number: 20843159.3
(22) Date of filing: 24.07.2020
(51) Int. Cl.: C12N 5/0775, C07K 14/475, A61K 35/28, A61P 25/00

(54) **METHOD FOR PREPARATION OF IMMORTALIZED STEM CELL LINE AND USE THEREOF**

(30) Priority: 24.07.2019 KR 20190089897
(71) Applicant: Slbigen Inc., Yeonsu-gu Incheon 21983 (KR)
(72) Inventor: LEE, Soon Min, Seoul 06762 (KR)
(74) Representative: Dehns
(86) International application number: PCT/KR2020/009769
(87) International publication number: WO 2021/015584

(57) **Abstract**

The present invention relates to a method for preparation of an immortalized stem cell line that has an immortalizing gene introduced thereinto and retains an expression potential of the introduced gene while being restrained from proliferation so that the immortalized stem cell line can be used as a cell therapeutic agent, and to a use thereof. The immortalized stem cell line of the present invention, which has undergone a radiation process, cannot proliferate while maintaining the expression of the introduced foreign protein at a certain level or higher and as such, can be advantageously used as clinical samples such as various cell therapeutic agents.

## Description

### [Technical Field]

### CROSS-REFERENCE TO RELATED APPLICATION

This application claims priority to and the benefit of Korean Patent Application No. 10-2019-0089897, filed on July 24, 2019, the disclosure of which is incorporated herein by reference in its entirety.

The present invention relates to a method for preparation of an immortalized cell line that has an immortalizing gene introduced thereinto and retains an expression potential of the introduced gene while being restrained from proliferation so that the immortalized stem cell line can be used as a cell therapeutic agent, and to a use thereof.

### [Background Art]

Treatment methods using cells are being developed around the world, and in particular, many studies are being conducted on cell therapeutic agents using adult stem cells. Mesenchymal stem cells (MSCs), which are adult stem cells, are multipotent cells capable of differentiating into bone, cartilage, muscle, fat, fibroblast, and the like. The MSCs can be obtained relatively easily from various adult tissues such as bone marrow, umbilical cord blood, and fat. MSCs have the specificity of moving to the site of inflammation or damage, and thus has a great advantage as a carrier for delivering therapeutic drugs. In addition, it can regulate the immune function of the human body by inhibiting or activating the functions of immune cells such as T cells, B cells, dendritic cells and natural killer cells. In addition, MSCs have the advantage that they can be cultured relatively easily *in vitro.* Due to these characteristics, studies for the use of MSC as a cell therapeutic agent are being actively conducted.

However, despite the advantages of MSCs, there are the following problems in producing MSCs that can be clinically used as cell therapeutic agents. First, there is a limit to the proliferation of MSCs, making it difficult to mass-produce them. Second, since the obtained MSCs are mixed with various types of cells, it is difficult to maintain the same effect during production. Third, when only MSC is used, the therapeutic effect is not high. Finally, there is a possibility that MSCs injected into the body can become cancer cells in the body.

Meanwhile, Korean Patent Registration No. 1585032 discloses a cell therapeutic agent containing mesenchymal stem cells cultured in a hydrogel. The patent document provides a composition that can be administered immediately by shortening the pretreatment process in the process of separating mesenchymal stem cells for use as a cell therapeutic agent, but does not mention at all about the problems of the mesenchymal stem cells as described above and methods for solving them. Therefore, there is a need for research on safe and effective mesenchymal stem cells that can be used as cell therapeutic agents.

The above information disclosed in this Background section is only for enhancement of understanding of the background of the invention and it may therefore contain information that does not form the prior art that is already known to a person of ordinary skill in the art.

### [Disclosure of Invention]

### [Technical Problem]

Accordingly, the present inventors have studied to develop a stem cell therapeutic agent that can be mass-produced and can maintain the same effect even in long-term culture, and as a result, completed the present invention by finding out the fact that when irradiating a specific range of radiation to stem cells into which immortalizing genes and foreign genes of the present invention are introduced, the expression of the protein by the introduced foreign genes is stably maintained, but the proliferation of the stem cells is suppressed.

That is, one aspect of the present invention is directed to providing a method for preparation of an immortalized stem cell line and the prepared immortalized stem cell line, the method comprising irradiating radiation to an immortalized stem cell line into which an immortalizing gene and a foreign gene are introduced.

Another aspect of the present invention is directed to providing a pharmaceutical composition using the prepared immortalized stem cells.

### [Technical Solution]

In one aspect, the present invention provides a method for preparation of an immortalized stem cell line, the method comprising: preparing a stem cell line by introducing an immortalizing gene; introducing a gene encoding a foreign protein into the stem cell line; cryopreserving the stem cell line; and irradiating radiation to the cryopreserved stem cell line.

In another aspect, the present invention also provides a pharmaceutical composition comprising the immortalized stem cell line produced by the above method as a cell therapeutic agent.

Hereinafter, the present invention will be described in more detail.

In an aspect, the present invention provides a method for preparation of an immortalized stem cell line, the method comprising: preparing a stem cell line by introducing an immortalizing gene; introducing a gene encoding a foreign protein into the stem cell line; cryopreserving the stem cell line; and irradiating radiation to the cryopreserved stem cell line.

The immortalized stem cell line of the present invention can maintain the characteristics of the stem cells even in long-term subculture by introducing an immortalizing gene, and has the characteristic that stable mass production is possible by maintaining the same quality even in mass production.

In particular, by introducing a step of irradiating radiation to the cryopreserved stem cell line during the production process, stem cells into which the immortalizing gene is introduced do not proliferate, and the introduced protein can be stably expressed for a long period of time, so it can be safely used as a cell therapeutic agent.

The immortalized stem cell line of the present invention can be prepared by the following method:
1) a step of introducing an immortalizing gene such as hTERT and/or c-Myc gene into a host cell;
2) a step of introducing a foreign gene into the host cell into which the immortalizing gene is introduced.

Various methods such as plasmid, retrovirus, lentivirus, AAV, etc. may be used for the gene introduction method in steps 1) and 2), and in the case of using a lentivirus, a vector containing a specific promoter for regulating the expression of a foreign gene may be additionally used when preparing a lentivirus containing a foreign gene, and in this case, it may include a step of further introducing a gene including the promoter.

Although not limited thereto, in an embodiment of the present invention, a gene was introduced into a cell using a lentiviral vector, and in this case, the TRE promoter whose expression is regulated with doxycycline or tetracycline was used, and to this end, the cells were further infected with a lentivirus containing the tTA gene.

The stem cells of the present invention are immortalized by introducing an immortalizing gene. As in step 1) above, in the present invention, the "immortalizing gene" may be an hTERT and/or c-Myc gene, but other genes that can be introduced as an immortalizing gene may be used without limitation.

As used herein, the term "c-Myc" refers to a gene encoding a transcription factor located on chromosome 8 of humans. The protein coded in c-Myc, which controls the expression of about 15% of intracellular genes, is a transcription regulatory factor, and when the transcription regulatory factor is overexpressed, cell activity and proliferation are promoted. In the present invention, "hTERT" refers to telomerase reverse transcriptase. The telomerase reverse transcriptase synthesizes DNA complementary to the RNA template of telomerase, forms an RNA-DNA hybrid, and becomes double-looped DNA and is inserted into the chromosome of the host cell. Then, in the host cell, instead of telomeres, telomerase attaches to chromosome telomeres and continues to produce telomeres, thereby making immortalized cells.

In addition, tTA, which may be further included in the above step, is a gene capable of regulating the expression of a target protein, and refers to a tetracycline transactivator. The Tet-off system used in the present invention can regulate the expression of a target protein according to the presence or absence of tetracycline or doxycycline in the same manner as described above.

As used herein, the "foreign gene" refers to a gene that is introduced into a stem cell and can encode a foreign protein. The immortalized stem cell line of the present invention has the characteristic of maintaining the expression potential of the protein encoded by the introduced foreign gene. In addition, the foreign protein is not limited in its type, and may be included without limitation as long as it can be introduced into stem cells and be expressed. Preferably, the foreign protein may be a protein having a therapeutic effect on a disease.

More specifically, the foreign protein may include, without limitation, growth factors such as hepatocyte growth factor (HGF), vascular epithelial growth factor (VEGF), nerve growth factor (NGF), brain-derived neurotrophic factor (BDNF), fibroblast growth factor (FGF), insulin-like growth factor (IGF), transforming growth factor (TGF), platelet-derived growth factor (PDGF), Bone morphogenetic protein (BMP), colony stimulating factor (CSF), epidermal growth factor (EGF), and keratinocyte growth factor (KGF); cytokines such as IL-2, IL-4, TNF, IF-γ, G-CSF, and GM-CSF; cancer therapeutic genes such as TNF-related apoptosis-inducing ligand protein (TRAIL) and/or cytosine deaminase (CD); or an antibody recognizing a specific antigen that can target cancer tissue, a chimeric antigen receptor (CAR) type antibody (mono or bi, tri-specific), as well as a chemokine receptor that promotes cell migration. In an embodiment of the present invention, an immortalized stem cell line introduced with BDNF, TRAIL and/or CD was prepared, but it is not limited thereto.

The immortalized stem cell line of the present invention may be one expressing BDNF. "Brain-derived neurotrophic factor (BDNF)" protein is the most abundantly distributed neurotrophin in the brain. It is known to promote the growth of neurons, and to regulate the synthesis, metabolism and release of neurotransmitters, and activity of neurons. In addition, it is known that BDNF protein is decreased in the prefrontal cortex or hippocampus of depressed patients, and can treat depression by increasing its concentration.

The BDNF protein according to the present invention may be a human-derived protein. The protein may be a polypeptide having the amino acid sequence of SEQ ID NO: 1. The BDNF protein may have about 80%, 90%, 95%, or 99% or more homology to the amino acid sequence of SEQ ID NO: 1. Meanwhile, the gene encoding the BDNF protein may be the nucleotide sequence of SEQ ID NO: 2. In addition, the nucleotide sequence encoding the BDNF protein may have about 80%, 90%, 95%, or 99% or more homology to the nucleotide sequence of SEQ ID NO: 2.

In addition, the immortalized stem cell line of the present invention may be one that expresses TRAIL and CD. "TNF-related apoptosis-inducing ligand (hereinafter TRAIL)" protein belongs to type 2 transmembrane cytokines in the TNF family. The TRAIL is one of the suicide genes, and selectively induces apoptosis of transformed cells. Specifically, TRAIL activates the apoptosis signaling system by binding to Death receptor-4 (DR-4), DR-5, decoy receptor or decoy receptor-2 present on the cell surface. TRAIL is not toxic to normal cells and is known to specifically induce apoptosis only in cancer cells.

The TRAIL protein according to the present invention may be a human-derived protein. The TRAIL protein exists in the form of a homotrimer capable of binding to three receptors. The TRAIL protein of the present invention may be a polypeptide having the amino acid sequence of SEQ ID NO: 3. The TRAIL protein may have about 70%, 80%, 90%, or 95% or more homology to the amino acid sequence of SEQ ID NO: 3. Meanwhile, the gene encoding the TRAIL protein may be a polynucleotide having the nucleotide sequence of SEQ ID NO: 4. In addition, the nucleotide sequence encoding the TRAIL protein may have about 70%, 80%, 90%, or 95% or more homology to the nucleotide sequence of SEQ ID NO: 4.

As used herein, the term "CD protein" is a cytosine deaminase protein, and may be in the form of "a fusion protein in which CD and UPRT (uracil phosphoribosyltransferase) are combined", and "CD protein" may be used interchangeably herein with "CD::UPRT". The sequence of the gene encoding the CD protein according to the present invention is a codon-optimized sequence by fusing the FCY1 gene encoding the CDase of Saccharomyces cerevisiae and the FUR1Δ105 gene encoding the UPRTase with 35 amino acids deleted from the N-terminus. The sequence may be those described in US Pat. No. 5,338,678, International Patent Publication No. WO 96/16183, and International Patent Publication No. WO 99/54481. According to an embodiment, the CD protein may be a polypeptide having the amino acid sequence of SEQ ID NO: 5. In addition, the CD protein may have about 70%, 80%, 90%, or 95% or more homology to the amino acid sequence of SEQ ID NO: 5. Meanwhile, the gene encoding the CD protein may be a polynucleotide having the nucleotide sequence of SEQ ID NO: 6. In addition, the nucleotide sequence encoding the CD protein may have about 70%, 80%, 90%, or 95% or more homology to the nucleotide sequence of SEQ ID NO: 6.

As used herein, the term "lentiviral vector" is a kind of retrovirus. The vector is also referred to as a lentiviral transfer vector interchangeably. The lentiviral vector is inserted into the genomic DNA of a cell to be infected to stably express the gene. In addition, the vector can transfer genes to dividing cells and non-dividing cells. Since the vector does not induce an immune response of a human body, its expression is continuous. In addition, it has an advantage that genes of a large size may be transferred as compared to an adenoviral vector, which is a viral vector used in the related art.

The recombinant lentiviral vector used in the present invention can regulate the expression of a gene loaded therein by a promoter. The promoter may be a cytomegalovirus (CMV), respiratory syncytial virus (RSV), human elongation factor-1 alpha (EF-1α) or tetracycline response elements (TRE) promoter. According to an embodiment, the recombinant lentiviral vector can regulate the expression of BDNF, TRAIL and CD proteins by one or two or more promoters. The promoter is operably linked to a gene encoding a protein to be expressed.

According to an embodiment of the present invention, the BDNF, TRAIL and/or CD proteins may be linked to a TRE promoter. It was confirmed that using other types of promoters without using the TRE promoter increased the doubling time as the passages passed, and the expression level of the foreign protein significantly decreased as the passages passed, and the cell morphology was changed.

The TRE promoter may activate transcription of a gene linked to a promoter by a tetracycline transactivator (tTA) protein. Specifically, when tetracycline or doxycycline is not present the tTA protein binds to the TRE promoter and activates transcription. On the other hand, when they are present, the tTA protein cannot bind to the TRE promoter and thus cannot activate transcription. Thus, the expression of BDNF, TRAIL and/or CD proteins may be regulated depending on the presence of tetracycline or doxycycline.

The term "operably linked" means that a particular polynucleotide is linked to another polynucleotide so that it can exert its function. The expression that a gene encoding a particular protein is operably linked to a promoter implies that the gene can be transcribed into mRNA by the action of the promoter and translated into a protein.

The "recombinant lentivirus" may be obtained by the steps of transforming a host cell with a lentiviral vector, packaging plasmid and envelope plasmid of the present invention; and separating the lentivirus from the transformed host cell.

The terms "packaging plasmid" and "envelope plasmid" are plasmids loaded with genes encoding proteins. These plasmids may provide, in addition to the lentiviral vector, the helper structures (e.g., plasm ids or isolated nucleic acids) necessary to produce the lentivirus. These structures contain elements useful for manufacturing and packaging the lentiviral vector in a host cell. Such elements may include structural proteins such as GAG precursors; processing proteins such as pol precursors; proteases, envelope proteins, and expression and regulatory signals necessary for production of proteins and production of lentivirus particles in host cells, and the like.

For the production of recombinant lentivirus, Clontech Laboratories' Lenti-X Lentiviral Expression System or Addgene's packaging plasmid (e.g., pRSV-Rev, psPAX, pCI-VSVG, pNHP, etc.) or envelope plasmid (e.g., pMD2.G, pLTR-G, pHEF-VSVG, etc.) may be used.

The term "transduction" refers to transferring a gene loaded in a recombinant lentiviral vector to a host cell through viral infection.

The host cell may be a human embryonic stem cell (hES), a bone marrow stem cell (BMSC), a mesenchymal stem cell (MSC), a human neural stem cell (hNSC), a limbal stem cell, or an oral mucosal epithelial cell. According to an embodiment of the present invention, the host cell may be a mesenchymal stem cell.

The term "mesenchymal stem cell (MSC)" refers to a multipotent stromal cell that can be differentiated into various cells including osteocytes, chondrocytes and adipocytes. Mesenchymal stem cells can be differentiated into cells of specific organs such as bone, cartilage, fat, tendon, nervous tissue, fibroblasts, and myocytes. These cells can be separated or purified from adipose tissue, bone marrow, peripheral nerve blood, umbilical cord blood, periosteum, dermis, mesodermal-derived tissue, and the like.

In an embodiment of the present invention, the immortalized mesenchymal stem cell line may be a cell line prepared so that expression of a foreign gene is suppressed by treatment with doxycycline *ex vivo* and the foreign gene is expressed by removal of doxycycline.

In an embodiment of the present invention, when the immortalized mesenchymal stem cell line is cultured in doxycycline-free medium (DMEM-low glucose, 10 ng/ml FGF, 10% FBS) under the conditions of 37°C and 5% CO₂ for 48 hours, it may be a cell line expressing the BDNF protein at an increased level of 4000 times or more, 3000 times or more, 1000 times or more, 500 times or more, 100 times or more, 10 times or more, 3 times or more, preferably 3 to 6000 times, 3 to 5000 times, 3 to 4000 times, 3 to 3000 times, 3 to 2000 times, 3 to 1000 times, 3 to 500 times, 3 to 300 times, 3 to 100 times, 30 to 6000 times, 30 to 4000 times, 30 to 3000 times, 30 to 1000 times, 300 to 6000 times, 300 to 3000 times or 300 to 1000 times or more, compared to doxycycline-treated medium from 1×10⁵ cells.

In an embodiment of the present invention, for the selection of immortalized mesenchymal stem cell line expressing the BDNF of the present invention, first, clones expressing 1.5 ng/ml or more of BDNF by the TRE promoter were selected when cultured in a 12 well plate for 48 hours after removal of doxycycline after monoclonal preparation, second, clones expressing less than 0.5 ng/ml of BDNF were selected when 1×10⁵ cells were treated with doxycycline in a 12 well plate and cultured for 48 hours, and then final clone candidates were selected after monoclonal preparation once more.

In an embodiment of the present invention, the immortalized mesenchymal stem cell line of the present invention may be a cell line in which the unique characteristics of mesenchymal stem cells are maintained by expressing CD44 and CD105 proteins, which are surface antigen proteins, even during long-term subculture. In addition, in an embodiment of the present invention, the immortalized mesenchymal stem cell line of the present invention may be a cell line in which the morphological characteristics of the mesenchymal stem cells are maintained since there is no change in the shape of mesenchymal stem cells even during long-term subculture up to 100 days.

The immortalized mesenchymal stem cell line of the present invention may be a cell line that can be mass-produced within a short time since it proliferates stably even during long-term subculture and its doubling time is as short as 5 to 25 hours, 5 to 20 hours, 5 to 15 hours, 10 to 25 hours, 10 to 20 hours or 10 to 15 hours. In an embodiment of the present invention, it was confirmed that the immortalized mesenchymal stem cell line of the present invention proliferated stably even during long-term subculture for 100 days or more, and the doubling time was short with 14 to 25 hours, with an average of 16 hours.

The immortalized mesenchymal stem cell line of the present invention is cryopreserved after production. The cryopreservation may be performed by a method known in the art, and is not particularly limited thereto. The cryopreservation temperature is not particularly limited, but may be in the range of -70 to -200°C. In an embodiment of the present invention, the cell line was stored in a liquid nitrogen tank, and the cryopreserved state was maintained even in the subsequent steps.

The immortalized mesenchymal stem cell line of the present invention is prepared including the step of irradiating radiation before being used as a cell therapeutic agent administered to a human body in order to suppress the cell proliferation that occurs when the immortalizing gene is introduced for mass production.

As used herein, the term "irradiating radiation or irradiation" is characterized in that using the principle that an ionization energy from a radiation source acts on highly sensitive DNA in mesenchymal stem cells, causing effective ionization, and cutting DNA chains, the irradiated mesenchymal stem cells lose their proliferative ability, but the expression of the introduced therapeutic gene is maintained during survival.

In the present invention, as long as the radiation can inhibit the proliferation of mesenchymal stem cells, it can use without limitation in types such as gamma rays, beta rays, neutron rays, X-rays, and electron beams.

In the present invention, the "irradiation dose" of the radiation refers to the amount of positive ion or electron energy generated while radiation such as X-rays or gamma rays in a certain place moves in the air. Accordingly, the irradiation dose exhibits difference in the dose actually absorbed by the subject according to the type of irradiation device, the environment to be irradiated, and the exposure time. Accordingly, the dose of radiation irradiated in the present invention was measured based on the "absorbed dose", which is a unit indicating the degree to which the energy of radiation is absorbed by the medium by the subject. That is, in the present invention, the range was measured by the amount of radiation energy absorbed by the immortalized stem cell line.

In an embodiment of the present invention, as immortalized stem cell lines, BM102 and BM01A cell lines into which BDNF was introduced and BM03 cell lines into which TRAIL and CD were introduced were subjected to irradiation tests, and as a result, a minimum absorbed dose that the immortalized stem cell line can be used as a cell therapeutic agent was measured by confirming that colony formation and cell number increase do not occur in cell lines that have absorbed radiation more than a certain absorbed dose.

That is, the absorbed dose in the present invention may be in a range more than at least 80 Gy, based on the stem cell line in which the radiation has been absorbed, preferably in a range more than 81 Gy, 82Gy, 83Gy, 84Gy, 85Gy, 86Gy, 87Gy, 88Gy, 89Gy, and 90Gy, and more preferably in a range of 80 Gy to 400 Gy absorbed dose. However, it was confirmed that even when irradiated with an absorbed dose exceeding the above range, the proliferation of stem cells did not occur and the expression of the foreign protein was maintained. The upper limit of the absorbed dose confirmed above is only an experimentally measured range, and does not mean that the upper limit is particularly limited. As confirmed from an embodiment of the present invention, the cell line of the present invention was suitable for use as a clinical sample even when irradiated with radiation of 500 Gy or more in terms of irradiation dose.

In addition, from an embodiment of the present invention, the range of absorbed dose absorbed by the stem cell line satisfies the following range:
90 to 110% of the irradiation dose when the irradiation dose is 0 to 100 Gy;
85 to 115% of the irradiation dose when the irradiation dose is 100 to 200 Gy; and
60~110% of the irradiation dose when the irradiation dose is 200 Gy or more.

When the above range is satisfied, the immortalized stem cell line of the present invention did not form colonies and increase the number of cells, and the expression level of the introduced foreign gene was maintained at a certain level or more. This is the result of confirming the safety of the stem cell line produced to be mass-produced by introducing the immortalizing gene.

In an embodiment of the present invention, the mesenchymal stem cell line produced by the above production process is an immortalized mesenchymal stem cell line transfected with a recombinant lentivirus containing a gene encoding a tetracycline response elements (TRE) promoter gene and a brain-derived neurotrophic factor (BDNF) protein, and the cell line provides an immortalized mesenchymal stem cell line in which the expression pattern of genes in the mesenchymal stem cell line cultured by removing doxycycline compared to the mesenchymal stem cell line cultured by treating with doxycycline *ex vivo* is shown as follows:
BDNF overexpression;
increased expression level of ANGPTL4, ANGPT1, CEND1, NFASC, GRAP2 or TRIM6 gene; and
reduced expression level of IL2RB, IL6, IL1B or PTGS2.

The immortalized mesenchymal stem cell line is an immortalized mesenchymal stem cell line in which BDNF expression is suppressed by treating doxycycline *ex vivo* and BDNF is expressed by removing doxycycline, and the transcriptome may be changed depending on whether BDNF is expressed or not.

As used herein, the term "transcriptome" refers to the sum of all RNAs expressed. The immortalized mesenchymal stem cell line of the present invention may include the following RNA changes compared to before and after doxycycline treatment.

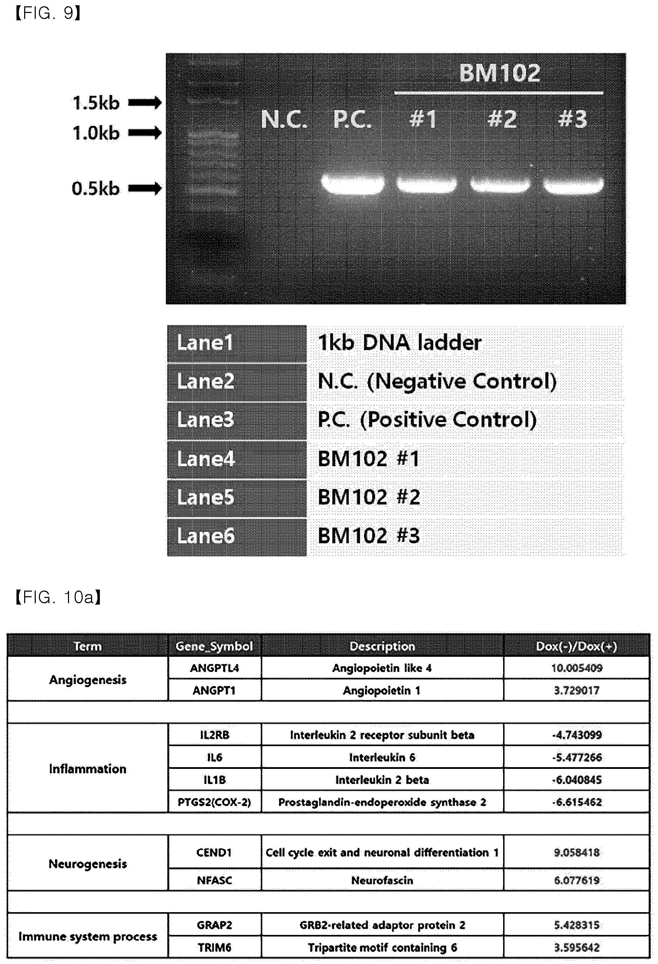

In an embodiment of the present invention, it was confirmed that when an immortalized mesenchymal stem cell line was grown in doxycycline-added medium and doxycycline was removed, BDNF was overexpressed, the expression of any one or more genes selected from the group consisting of ANGPTL4, ANGPT1, CEND1, NFASC, GRAP2 and TRIM6 was increased, and the expression of IL2RB, IL6, IL1B or PTGS2 genes was decreased (FIG. 10a).

In the present invention, ANGPLT4 (angiopoietin like 4) and ANGPT1 (angiopoietin 1) are known to be associated with angiogenesis process; IL2RB (interleukin 2 receptor subunit beta), IL6 (interleukin 6), IL1B (interleukin 1 beta) and PTGS2 (prostaglandin-endoperoxidase synthase 2) are associated with inflammation; CEND1 (cell cycle and neuronal differentiation 1) and NFASC (neurofascin) are associated with neurogenesis process; and GRAP2 (GRB2-related adapter protein 2) and TRIM6 (Tripartite motif containing 6) are known to be associated with the immune system.

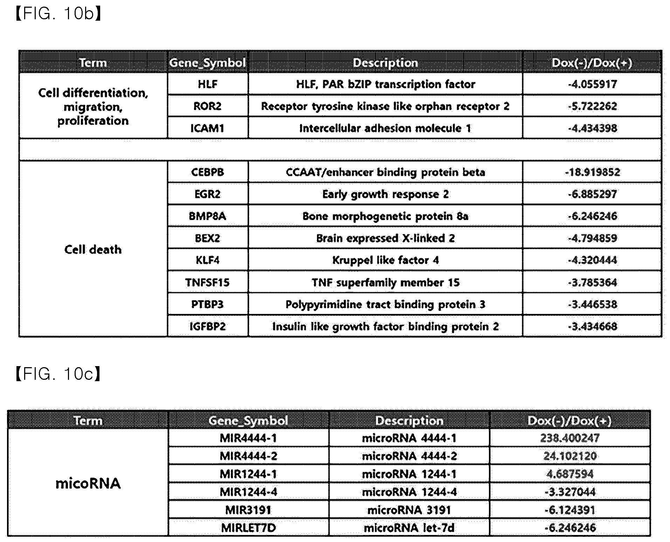

In an embodiment of the present invention, it was further confirmed that when the immortalized mesenchymal stem cell line was grown in doxycycline-added medium and doxycycline was removed, the expression level of HLF, ROR2, or ICAM1 gene was decreased, and the expression level of CEBPB, EGR2, BMP8A, BEX2, KLF4, TNFSF15, PTBP3 or IGFBP2 gene was decreased (FIG. 10b).

HLF (HLF, PAR bZIP transcription factor), ROR2 (Receptor tyrosine kinase like orphan receptor 2) and ICAM1 (Intracellular adhesion molecule 1) genes of the present invention are known as genes that regulate cell differentiation, migration and proliferation.

In addition, CEBPB (CCAAT/enhancer binding protein beta), EGR2 (early growth receptor 2), BMP8A (Bone morphogenetic protein 8a), BEX2 (brain expressed X-linked 2), KLF4 (kruppel like factor 4), TNFSF15 (TNF superfamily member 15), PTBP3 (polypyrimidine tract binding protein 3) and IGFBP2 (insulin growth factor binding protein 2) are known as genes that regulate apoptosis.

In an embodiment of the present invention, it was further confirmed that when the immortalized mesenchymal stem cell line was grown in doxycycline-added medium and doxycycline was removed, the expression of MiR4444-1, MiR4444-2, and MiR1244-1 was increased, and the expression of MiR1244-4, MiR319l, and MiRLET7D was decreased (FIG. 10c).

In another aspect, the present invention provides a pharmaceutical composition comprising the immortalized mesenchymal stem cell line prepared by the method as described above. The pharmaceutical composition may include, as an active ingredient, a protein expressed from a foreign gene introduced into an immortalized mesenchymal stem cell line.

The pharmaceutical composition may further comprise a pharmaceutically acceptable carrier. The carrier is commonly used in the manufacture of pharmaceuticals, and may comprise lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, and the like.

In addition, the pharmaceutical composition of the present invention may further comprise a pharmaceutically acceptable additive selected from the group consisting of a lubricant, a wetting agent, a sweetening agent, a flavoring agent, an emulsifying agent, a suspending agent, a preservative, and a combination thereof.

The carrier may be comprised in an amount of about 1% to about 99.99% by weight, preferably about 90% to about 99.99% by weight, based on the total weight of the pharmaceutical composition of the present invention, and the pharmaceutically acceptable additive may be comprised in an amount of about 0.1% to about 20% by weight.

The pharmaceutical composition may be prepared in a unit dosage form by formulating using a pharmaceutically acceptable carrier and excipient, or may be prepared by filling into a multi-dose container, according to a conventional method. In this case, the formulation may be in the form of a solution, suspension, syrup or emulsion in oil or aqueous medium, or may be in the form of an extract, powder, granule, tablet or capsule, and may additionally contain a dispersing or stabilizing agent.

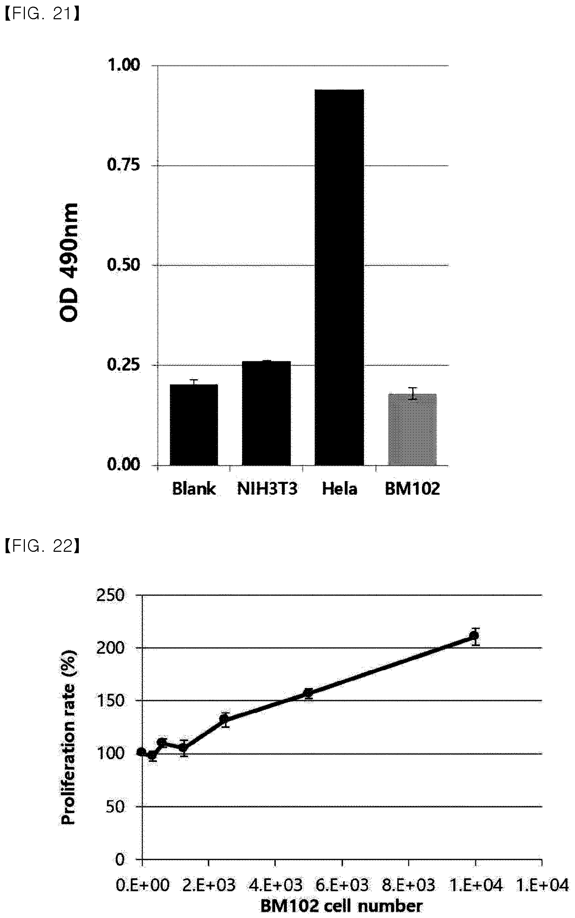

In an embodiment of the present invention, when C6 glial cells, known to grow well by BDNF, were co-cultured with the BM102 cell line, it was confirmed that since the proliferation rate of C6 cells increases in proportion to the number of BM102 cell lines, the BM102 cell line can stably mass-produce BDNF enough to be used as a cell therapeutic agent and the BM102 cell line stably expressing BDNF can be used as a cell therapy to protect or treat nerve cells (FIG. 22).

In addition, in an embodiment of the present invention, it was confirmed that the BM102 cell line had no transforming ability during cell culture and no *in vitro* tumorigenicity (FIG. 21).

Accordingly, since the immortalized mesenchymal stem cell line of the present invention has no tumorigenicity despite being a transformed cell into which BDNF and immortalizing genes are introduced, and can exhibit a neuroprotective effect through the expression of BDNF, it can be safely used in the treatment of various central nervous system disorders.

The neurological disease may be selected from the group consisting of Alzheimer's disease (AD), Parkinson's disease (PD), amyotrophic lateral sclerosis (ALS), cerebral infarction, chronic brain injury, ischemic brain disease, spinal cord injury, Huntington's disease (HD), Rett's disease (RD), stroke, multiple sclerosis, traumatic brain injury, neonatal hypoxic ischemic encephalopathy, and erectile dysfunction.

In addition, the present invention provides a method for preventing or treating a neurological disease as described above, comprising administering the pharmaceutical composition of the present invention to a subject.

The subject may be a mammal, specifically a human. The administration route and dosage of the pharmaceutical composition may be adjusted in various ways and amounts for administration to a subject depending on the condition of a patient and the presence or absence of a side effect, and the optimal administration method and dosage may be selected by a person skilled in the art in a suitable range. In addition, the pharmaceutical composition may be administered in combination with other drugs or physiologically active substances known to have a therapeutic effect on a disease to be treated, or may be formulated in the form of a combination formulation with other drugs.

When the pharmaceutical composition is administered parenterally, examples of administration include subcutaneous, ocular, intraperitoneal, intramuscular, oral, rectal, intravitreal, intracerebral, intracranial, intraspinal, intraventricular, intrathecal, intranasal, intravenous and arterial including carotid artery administrations.

The administration may be administered 1 or more times, 2 or more times, 3 or more times, 4 or more times, 1 to 4 times or more, and specifically in two divided doses. In the case of repeated administrations, it may be administered at intervals of 12 to 48 hours, 24 to 36 hours, 1 week, 2 weeks to 4 weeks, and specifically, may be administered at intervals of 24 hours or 1 week or more. The administration may be conducted in an amount of 1.0×10⁶ to 1.0×10¹² TU, specifically 1.0×10⁸ to 1.0×10¹⁰ TU per day for adults in the case of lentiviruses. Meanwhile, the administration may be conducted in an amount of 1.0×10⁵ to 1.0x10¹¹ cells, specifically 1.0×10⁷ to 1.0×10⁹ cells per day for adults in the case of cells. In the case of a large dose, it may be administered several times a day.

### [Advantageous Effects]

The method for preparation of an immortalized stem cell line of the present invention further comprises the step of irradiating radiation to a stem cell line into which an immortalizing gene and a foreign gene is introduced, and accordingly can produce an immortalized stem cell line in which the expression level of the foreign protein that can be used as a therapeutic ingredient is maintained for a long period of time while having high safety due to low possibility of abnormal differentiation and proliferation. In addition, the immortalized stem cell line prepared according to the above preparation method can be usefully used as a pharmaceutical composition for the prevention or treatment of various diseases.

### [Brief Description of Drawings]

The above and other aspects, features, and advantages of the present invention will become more apparent to those of ordinary skill in the art by describing embodiments thereof in detail with reference to the accompanying drawings, in which:
FIG. 1 is a graph comparing cell proliferation rates of MSCs immortalized by c-Myc (STEM24) and non-immortalized MSCs (BM-MSCs):
   X-axis: culture period; and
   Y-axis: cumulative population doubling level (PDL).
FIG. 2 is a graph comparing cell proliferation rates of immortalized MSCs introduced with c-Myc and hTERT and non-immortalized MSCs:
   imMSC: immortalized MSC;
   MSC: non-immortalized MSC;
   X-axis: culture period; and
   Y-axis: cumulative population doubling level (PDL).
FIG. 3 is a graph showing the expression level of BDNF protein according to the presence or absence of doxycycline in clones whose expression level of BDNF protein was confirmed, in which FIG. 3a shows the expression level of the clones for selecting BM102 of the present invention and FIG. 3b shows the expression level of the clones for selecting BM01A of the present invention.
FIG. 4 shows the comparison of the expression of surface antigen proteins CD44 and CD105, which are unique characteristics of MSCs after genetic engineering of the BM102 monoclonal cell line, and the expression of surface antigen protein of bone marrow-derived MSCs
FIG. 5 is a graph confirming the proliferation rate while culturing immortalized MSC cells transduced with a lentivirus containing the BDNF gene, for a long time:
   X-axis: culture period; and
   Y-axis: cumulative population doubling level (PDL).
FIG. 6 is a graph confirming the cell proliferation rate of BM102 according to the presence or absence of doxycycline treatment during long-term culture:
   X-axis: culture period; and
   Y-axis: cumulative population doubling level (PDL).
FIG. 7 is a view confirming that morphological characteristics of cells are maintained for each passage of the BM102 cell line depending on the presence or absence of doxycycline treatment.
FIG. 8 is a graph showing the expression level of BDNF protein in the BM102 cell line depending on the presence or absence of doxycycline treatment.
FIG. 9 is a view confirming that the gene was introduced into the BM102 cell line.
FIG. 10 shows the changing genes by comparing the transcriptome of the BM102 cell line depending on the presence or absence of doxycycline treatment.
FIG. 10a is a diagram showing transcriptome changes of genes known to be involved in angiogenesis, inflammation, neurogenesis and immune system processes in the BM102 cell line according to the removal of doxycycline. An increased transcriptome value was indicated as a positive value, and a decreased transcriptome value was indicated as a negative value.
FIG. 10b is a diagram showing transcriptome changes of genes known to be involved in cell differentiation, migration, proliferation and apoptosis in the BM102 cell line according to the removal of doxycycline. An increased transcriptome value was indicated as a positive value, and a decreased transcriptome value was indicated as a negative value.
FIG. 10c is a diagram showing transcriptome changes of micro RNA genes in the BM102 cell line according to the removal of doxycycline. An increased transcriptome value was indicated as a positive value, and a decreased transcriptome value was indicated as a negative value.
FIG. 11 shows a result of measuring the number of cells after irradiating gamma rays to the BDNF-expressing immortalized stem cell line (BM102).
FIG. 12 shows a result of measuring the number of cells after irradiating X-rays to the BDNF-expressing immortalized stem cell line (BM102).
FIG. 13 shows a result of confirming the change in cell titer through BDNF expression level after irradiating gamma rays to the BDNF-expressing immortalized stem cell line (BM102).
FIG. 14 shows a result (primary) of confirming the change in cell titer through BDNF expression level after irradiating X-rays to the BDNF-expressing immortalized stem cell line (BM102).
FIG. 15 shows a result (secondary) of confirming the change in cell titer through BDNF expression level after irradiating X-rays to the BDNF-expressing immortalized stem cell line (BM102).
FIG. 16 shows a result of measuring the number of cells after irradiating gamma rays to the BDNF-expressing immortalized stem cell line (BM01A).
FIG. 17 shows a result of confirming the change in cell titer through BDNF expression level after irradiating gamma rays to the BDNF-expressing immortalized stem cell line (BM01A).
FIG. 18 shows a result of measuring the number of cells after irradiating low-level gamma rays to the TRAIL and CD-expressing immortalized stem cell line (BM03).
FIG. 19 shows a result of measuring the number of cells after irradiating high-level gamma rays to the TRAIL and CD-expressing immortalized stem cell line (BM03).
FIG. 20 shows a result of confirming the change in cell titer through the TRAIL and CD gene expression levels after irradiating low-level gamma rays to the TRAIL and CD-expressing immortalized stem cell line (BM03).
FIG. 21 is a graph confirming that there is no tumorigenicity even after genetic engineering of the BM102 cell line.
FIG. 22 is a graph confirming the proliferation rate of C6 cells when the BM102 cell line and C6 cells are co-cultured:
   X-axis: number of BM102 cells; and
   Y-axis: proliferation rate of C6 cells.

### [Best Mode for Carrying Out the Invention]

In an aspect, the present invention relates to a method for preparation of an immortalized stem cell line, the method comprising: preparing a stem cell line by introducing an immortalizing gene, introducing a gene encoding a foreign protein into the stem cell line, cryopreserving the stem cell line, and irradiating radiation to the cryopreserved stem cell line.

As an embodiment of the present invention, the immortalizing gene is c-Myc and/or hTERT.

As an embodiment of the present invention, the foreign protein is selected from the group of growth factors, cytokines or cancer therapeutic proteins, antibodies, and chemokine receptors.

As an embodiment of the present invention, the immortalized stem cell line is a human embryonic stem cell (hES), a bone marrow stem cell (BMSC), a mesenchymal stem cell (MSC), a human neural stem cell (hNSC), a limbal stem cell, or an oral mucosal epithelial cell.

As an embodiment of the present invention, the radiation is irradiated so that the absorbed dose to the stem cell line is at least 80 Gy, and more specifically, the absorbed dose satisfies the range of: 80 to 140% of the irradiation dose when the irradiation dose is 0 to 100 Gy; 80 to 140% of the irradiation dose when the irradiation dose is 100 to 200 Gy; and 60 to 140% of the irradiation dose when the irradiation dose is 200 Gy or more.

As an embodiment of the present invention, the radiation is selected from the group of gamma rays, beta rays, neutron rays, X-rays and electron beams.

In another aspect, the present invention relates to a pharmaceutical composition comprising an immortalized stem cell line prepared by the above method.

As an embodiment of the present invention, the pharmaceutical composition comprises an immortalized stem cell line expressing brain-derived neurotrophic factor (BDNF).

As an embodiment of the present invention, the pharmaceutical composition has a preventive or therapeutic effect on neurological diseases, and the neurological disease is selected from the group consisting of Alzheimer's disease (AD), Parkinson's disease (PD), amyotrophic lateral sclerosis (ALS), cerebral infarction, chronic brain injury, spinal cord injury, Huntington's disease (HD), Rett's disease (RD), ischemic brain disease, stroke and traumatic brain injury, neonatal hypoxic ischemic encephalopathy, and multiple sclerosis.

In yet another aspect, the present invention relates to a use of an immortalized stem cell line prepared by the above method as a cell therapeutic agent.

In yet another aspect, the present invention relates to a method for treating a disease by administering an immortalized stem cell line prepared by the above method in a therapeutically effective amount to a subject in need thereof.

### [Mode for Carrying Out the Invention]

Hereinafter, the present invention is described in detail with reference to the following Examples. However, the following Examples are only intended to illustrate the present invention, and the present invention is not limited thereto.

### Example 1. Preparation of immortalized mesenchymal stem cells (MSCs)

### Example 1.1. Preparation of lentiviral vector containing immortalizing gene

To immortalize MSCs, lentiviral vectors containing immortalizing genes c-Myc and/or hTERT were prepared. In this case, a gene construct expressing a tTA protein was also inserted to use a Tet-off system.

First, a pBD lentiviral vector was prepared by substituting an EF promoter in an expression cassette of a pWPT vector (Bioneer, synthesized) with a CMV promoter. The c-Myc gene (SEQ ID NO: 7) was inserted into the pBD lentiviral vector so that the expression could be regulated by the CMV promoter. The vector prepared above was named pBD-1.

Meanwhile, the hTERT gene (SEQ ID NO: 8) was inserted into the pBD lentiviral vector so that the expression could be regulated by the CMV promoter. A gene having resistance to zeomycin (ZeoR; SEQ ID NO: 12) was inserted thereinto so that the expression could be regulated by an RSV promoter. The vector prepared above was named pBD-2.

In addition, the tTA (tetracycline transactivator) gene (SEQ ID NO: 9) was inserted into the pBD lentiviral vector so that the expression could be regulated by the CMV promoter. The vector prepared above was named pBD-3.

### Example 1.2. Production of lentivirus containing immortalizing gene

Using the lentiviral vectors prepared in Example 1.1, lentiviruses containing an immortalizing gene were produced by the following method.

First, Lenti-X cells (Clontech Laboratories, USA) were cultured in a 150 mm dish using DMEM medium containing 10% fetal bovine serum. Meanwhile, the lentiviral vectors were extracted and quantified from DH5α E. coli cells using the EndoFree Plasmin Maxi Kit (Qiagen, USA).

After the cultured Lenti-X cells were washed with PBS, 3 ml of TrypLE^{™} Select CTS^{™} (Gibco, USA) was added. After leaving the cells at 37°C for about 5 minutes, it was confirmed that the cells were detached. The detached cells were neutralized by adding 7 ml of DMEM medium containing 10% fetal bovine serum. The neutralized cells were collected in a 50 ml tube and centrifuged at 1,500 rpm for 5 minutes. The cells were re-suspended by removing the supernatant and adding 10 ml of DMEM culture medium containing 10% fetal bovine serum. Suspended cells were counted with a hematocytometer, and then aliquoted into 1.2×10⁷ cells in a 150 mm dish. When the aliquoted cells were cultured to reach about 90% confluence, the cells were transduced with a mixture of 12 µg of lentiviral vector, 12 µg of psPAX (Addgene; gag-pol expression, packaging plasmid) and 2.4 µg of pMD.G plasmid (Addgene; VSVG expression, envelope plasmid). Lipofectamine (Invitrogen, USA) and Plus Reagent (Invitrogen, USA) were used to aid in transduction. After 6 hours of transduction, the medium was exchanged with DMEM containing 10% fetal bovine serum. After the cells were cultured for an additional 48 hours, the supernatant was collected.

The obtained supernatant was mixed with a lentivirus concentration kit (Lenti-X concentrator, Clontech Laboratories, USA), and then cultured at 4°C overnight. A virus was obtained by centrifuging the supernatant under the conditions of 4°C and 4,000 rpm for 2 hours, and the virus was re-suspended in 0.5 ml of DMEM without FBS.

### Example 1.3. Preparation of immortalized mesenchymal stem cells

Immortalized MSCs were prepared using the lentiviruses containing the immortalizing genes produced in Example 1.2.

First, bone marrow-derived MSCs were prepared by the following method. Specifically, a bone marrow aspirate was obtained from the iliac crest of a healthy donor. The bone marrow aspirate was mixed with 20 IU/ml heparin in a sterile container to inhibit coagulation. After the bone marrow mixture was centrifuged under the conditions of 4°C and 739 G for 7 minutes, the supernatant was removed and was mixed with 10-fold volumes of sterilized water. A pellet of cells was obtained by centrifuging the mixture again under the same conditions. The obtained pellet was suspended in DMEM-low glucose (11885-084, Gibco, USA) medium containing 20% fetal bovine serum and 5 ng/ml b-FGF (100-18B, Peprotech, USA) and aliquoted into a culture flask. After the aliquoted one was cultured under the conditions of 37°C, and 5% CO₂ for 24 to 48 hours, the medium was exchanged with a new medium. The cultured one was subcultured while exchanging the medium with a new medium every 3 to 4 days, and after 2 weeks of culture, it was checked whether MSCs were present using a fluorescence cell analyzer.

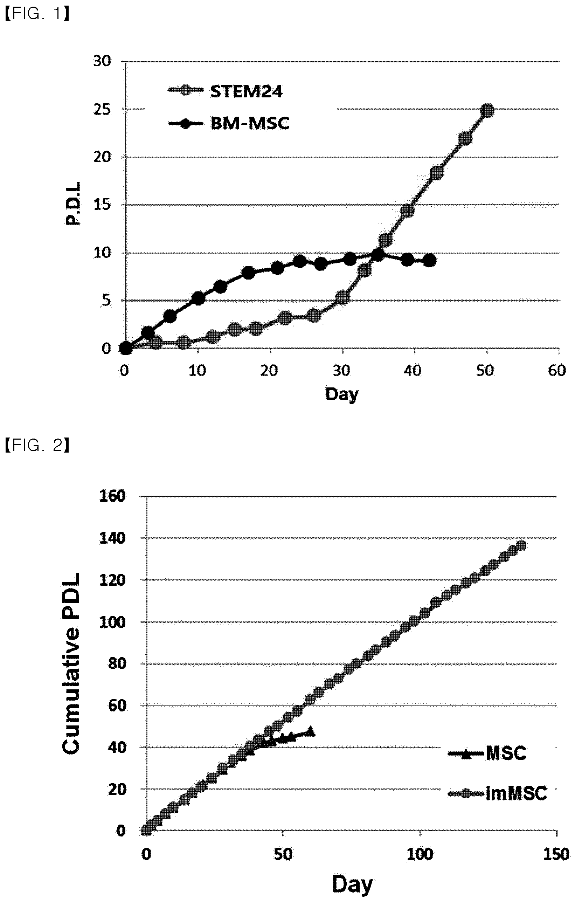

The prepared MSCs were infected with the pBD-1 lentivirus produced in Example 1.2 at 100 MOI using Retronectin (Clontech Laboratories, USA). As a result, the cell proliferation rates of MSCs containing c-myc and MSCs not containing c-myc are shown in FIG. 1. As shown in FIG. 1, MSC cells infected with the lentivirus containing the immortalizing gene c-Myc, as immortalized by the immortalizing gene, had an increased proliferation rate based on 30 days of culture after infection, and maintained a high proliferation rate even up to 50 days. In contrast, the cell proliferation rate of normal MSC cells decreased sharply after 20 days of culture.

In addition, cells infected with the lentivirus containing c-Myc were infected with the pBD-2 lentiviral vector containing hTERT at 100 MOI. After infection, 500 µg/ml of zeomycin was added to the culture medium of the stabilized cells to select pBD-2 lentivirus-infected cells.

In this regard, as shown in FIG. 2, MSC cells infected with a lentivirus containing both c-Myc and hTERT, which are immortalizing genes, maintained a high cell proliferation rate even after 120 days of culture. In contrast, the cell proliferation rate of normal MSC cells decreased sharply after 40 days of culture.

In addition, cells infected with the pBD-1 and/or pBD-2 were infected with the pBD-3 lentiviral vector containing tTA at 100 MOI. After infection, 1 µg/ml of puromycin was added to the culture medium of the stabilized cells to select pBD-3 lentivirus-infected cells.

### Example 2. Preparation of lentivirus containing foreign gene

### Example 2.1. Preparation of lentivirus containing BDNF gene

A BDNF gene (SEQ ID NO: 2) was inserted into the pBD lentiviral vector prepared in Example 1.1.

First, in order to confirm the effect of a promoter on BDNF expression, a lentiviral vector was prepared to express the BDNF gene using a CMV promoter and a TRE promoter, respectively and MSCs expressing the BDNF gene were prepared using the lentiviral vector. As a result, it was confirmed that in the case of a cell line to which the CMV promoter was applied, the MSCs expressing BDNF were morphologically changed during long-term subculture, and the expression rate of BNDF decreased as the subculture progressed.

Accordingly, the BNDF gene was inserted into each pBD vector so that expression was regulated by the TRE promoter. The TRE promoter can regulate the expression of the gene linked to the promoter depending on whether doxycycline is added or not.

In addition, using the lentiviral vectors pBD-4 and pBD-5 containing the BDNF gene prepared above, lentiviruses were produced in the same manner as described in Example 1.2. The produced lentivirus was prepared at a concentration of 4.7×10⁶ copies/ml (pBD-4) and 1.4×10¹² copies/ml (pBD-5), respectively.

### Example 2.2. Preparation of lentivirus containing TRAIL and CD genes

Lentiviruses containing TRAIL and CD genes were prepared according to the contents described in Korean Patent No. 10-1985271. TRAIL gene (SEQ ID NO: 4) and CD gene (SEQ ID NO: 6) were inserted into the pBD lentiviral vector prepared in Example 1.1. In this case, the inserted TRAIL and CD genes were linked to an internal ribosome entry site (IRES) such that expression is regulated by the TRE promoter. The IRES is a ribosome binding site, allowing translation to start even without a 5'-cap structure, so that two proteins can be expressed in one mRNA. Meanwhile, the TRE promoter can regulate the expression of the gene linked to the promoter depending on whether doxycycline is added or not.

The vector into which the TRAIL and CD were inserted was named pBD-6, and using this, a lentivirus was produced in the same manner as described in Example 1. The produced lentivirus was prepared at a concentration of 7.6×10⁸ TU/ml.

### Example 3. Preparation of immortalized stem cells transfected with lentivirus containing foreign gene

### Example 3.1. Preparation of MSCs transfected with lentivirus containing BDNF gene

### 3.1.1. Preparation of BM102 cell line

The immortalized MSC prepared in Example 1.3 was infected with the lentivirus containing the BDNF gene produced in Example 2.1 at 100 MOI to prepare cells expressing the BDNF gene. Here, the lentivirus produced by the pBD-1 vector into which among immortalizing genes c-Myc was introduced was used. The transduced cells were cultured in a medium added with 2 µg/ml doxycycline (631311, Clontech, USA) to suppress the expression of BDNF protein during culture.

The cells were cultured to form colonies using a clone select imager. After numbering each well inoculated with cells in a 96-well plate, the presence or absence of BDNF protein expression was confirmed with the human BDNF DuoSet ELISA kit (R&D systems, DY248, USA) among about 200 clones that confirmed colony formation.

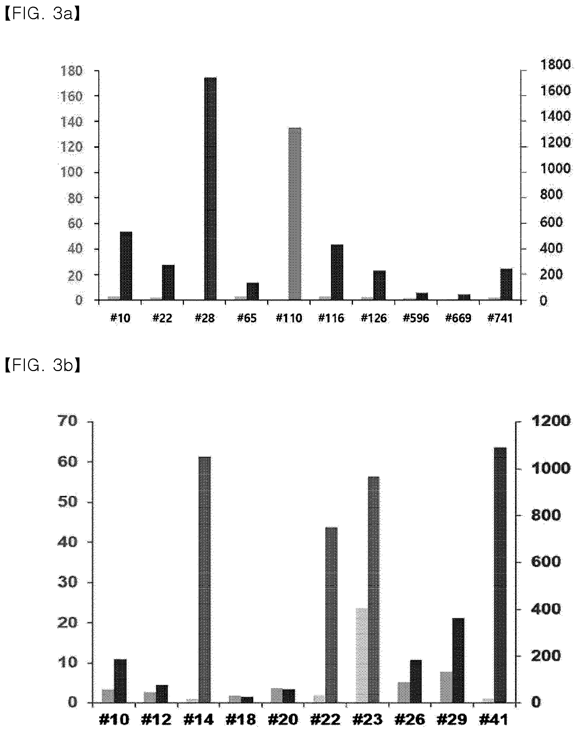

Among the cells, for immortalized MSCs prepared by lentivirus introduced with pBD-4, selected were clones #10, #22, #28, #65, #110, #116, #126, #596, #669 and #741 expressing BDNF protein of 1.5 ng/ml or more following doxycycline removal. Thereafter, clones #28 and #110 expressing BDNF protein at 0.5 ng/ml or less upon doxycycline treatment were selected (FIG. 3a).

As a result of confirming the cell proliferation ability of the selected clones, clone #28 showing a stable proliferation pattern and the best BDNF protein expression level was named BM102 cell line and deposited as a patent strain (Korea Research Institute of Bioscience and Biotechnology, KCTC13876BP).

### 3.1.2. Preparation of BM01A cell line

In addition, in the same manner as in 3.1.1 above, immortalized MSCs infected with lentiviruses introduced with pBD-1 and pBD-2 were prepared. The method for preparation of the cell line was according to the method described in Korean Patent No. 10-2074336. After virus infection, after stabilizing the cells, 500 µg/ml of G418 was added to the culture medium to select cells infected with pBD-5 lentivirus into which both c-Myc and hTERT genes were introduced. The selected cells were cultured in a medium added with 1 µg/ml doxycycline (631311, Clontech, USA) to suppress the expression of BDNF protein during culture.

The selected cells were cultured to form colonies. By confirming the presence or absence of BDNF protein expression with human BDNF DuoSet ELISA kit (R&D systems, DY248, USA), among the clones #1 to #50 formed, clones #10, #12, #14, #18, #20, #22, #23, #26, #29 and #41 expressing BDNF protein were selected, and clones #14, #22, #23 and #41 not expressing BDNF protein upon doxycycline treatment were reselected (FIG. 3b). Cell proliferation ability was confirmed by the same method, and clone #41 showing a stable proliferation pattern and the highest expression level was named BM-01A cell line.

### Example 3.2. Preparation of MSCs transfected with lentiviruses containing TRAIL and CD genes

According to the method described in Korean Patent No. 10-1985271, the immortalized MSC prepared in Example 1-3 was infected with a lentivirus containing the TRAIL and CD genes produced in Example 2-2 to prepare cells expressing TRAIL and CD genes. Infection was performed in the same manner as described in Examples 1-3. After infection, 1 µg/ml of doxycycline (631311, Clontech, USA) was added to the stabilized cell culture medium and cultured in a state in which the expression of TRAIL and CD was suppressed. After the cells were stabilized, the cells were cultured for 72 hours in a culture medium without doxycycline to induce expression of TRAIL and CD, and FACS was performed with the cells to select cells expressing TRAIL on the cell surface.

Specifically, the cells infected with the lentivirus containing the TRAIL and CD genes were aliquoted to 5×10⁵ per FACS tube, and the supernatant was removed by centrifugation at 4°C at 1,500 rpm for 5 minutes. To this, 1 ml of FACS buffer (PBS containing 2% fetal bovine serum) was added to resuspend the cells, and centrifuged under the same conditions to remove the supernatant. After performing the above washing procedure once more, the cells were resuspended in 1 ml of FACS buffer. A mixture of 0.3 µl LIVE/DEAD^{®} Fixable Near-IR Dead Cell Stain (Life Technologies-Molecular Probes, USA) and 5 µl APC anti-human CD253 antibody, which is an anti-TRAIL antibody, (BioLegend, Cat#. 308210, USA) added in 200 µl FACS buffer was added to the resuspended cells, and reacted at 4°C for 30 minutes. After the reaction, the cells were washed twice in the same manner as above, and the supernatant was removed. 300 µl of fixing buffer (PBS containing 2% formaldehyde and 1% fetal bovine serum) was added to the washed cells, and the cells were left at 4°C for at least 15 minutes. The cells were analyzed with a FACS instrument (LSRFortessa, BD biosciences, USA), and cells expressing TRAIL were selected and cultured to form colonies.

A cell line was established by culturing monoclonal cells from the formed colonies, and this was named BM-03.

### Example 4. Irradiation test for immortalized stem cell line

In order to use each immortalized MSC cell line prepared in Example 3 as a clinical sample, a irradiation step was additionally performed to maintain the expression level of the therapeutic protein without cell proliferation, and through this, a suitable irradiation (absorbed) dose was confirmed.

First, the immortalized MSC cell line formulated and cryopreserved in a liquid nitrogen tank was transported using a mobile cartridge prepared for irradiation. In this case, the cryopreserved was maintained using dry ice.

Irradiation was performed on the cryopreserved immortalized MSC cell line. Irradiation was carried out using a known method and device, and for gamma rays, a low level gamma ray irradiation device or a high level gamma ray irradiation device (MDS Nordion, Canada) was used, and for X-rays, X-RAD 320 X-RAY IRRADIATOR (Seoul National University Graduate School of Convergence Science and Technology) or RS1800Q (Rad Source technologies, Inc) was used. Even during irradiation, the temperature inside the mobile cartridge was maintained at a temperature that could sustain the cryopreserved by dry ice, and the irradiation-completed MSC cell line was stored again in a liquid nitrogen tank.

In order to confirm the irradiation dose, the radiation absorbed dose was measured for each dose using a separate alanine dosimeter, and the absorbed dose of the immortalized MSC cell line could be calculated from the above results.

After thawing 3 vials for each specimen of the irradiation-completed MSC cell line for about 2 minutes in a 37°C constant-temperature water bath, 9 mL of PBS was added and centrifuged for 5 minutes at 4°C and 1,500 rpm, and then the supernatant was removed, and the cells was suspended in DMEM-low glucose (11885-084, Gibco, USA) medium containing 10% FBS (16000-044, Gibco, USA) and 10 ng/mL b-FGF (100-18B, Peprotech, USA). The number of suspended cells was measured with a hematocytometer, and then the experiment was performed.

### Experimental Example 1. Confirmation of surface antigen protein expression in BM102 cell line

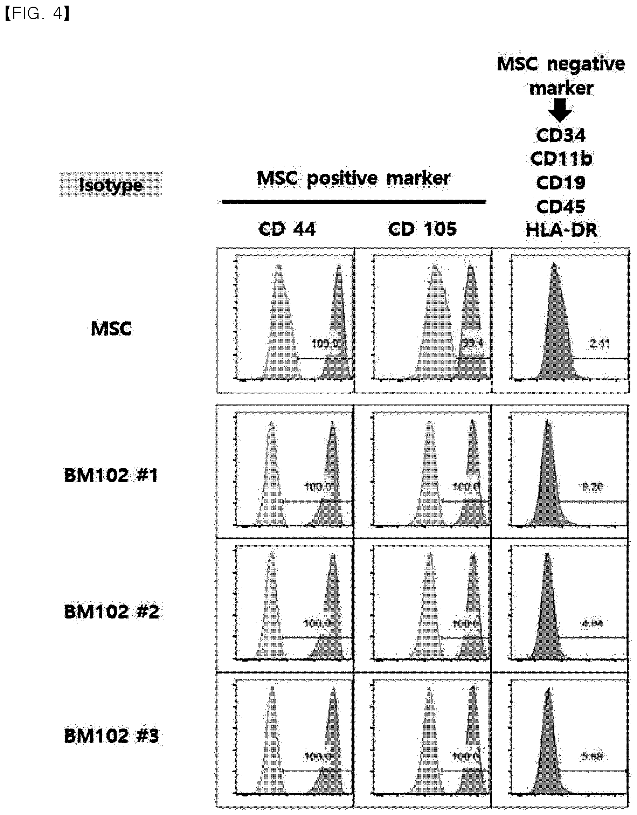

The expression of surface antigen proteins of the bone marrow-derived MSC before gene insertion and the BM102 cell line in which the BDNF gene was inserted was analyzed using a human MSC analysis kit (Stemflow^{™}, Cat No. 562245, BD). The experiment was performed according to the manual included in each kit, and the experimental results are shown in FIG. 4.

As a result, as shown in FIG. 4, it was demonstrated that even after the BM102 cell line was subjected to genetic engineering to express BDNF, the expression of the surface antigen proteins CD44 and CD105, which are the unique characteristics of MSCs, was similar to those of the bone marrow-derived MSCs.

### Experimental Example 2. Confirmation of proliferation rate of BM102 cell line before irradiation

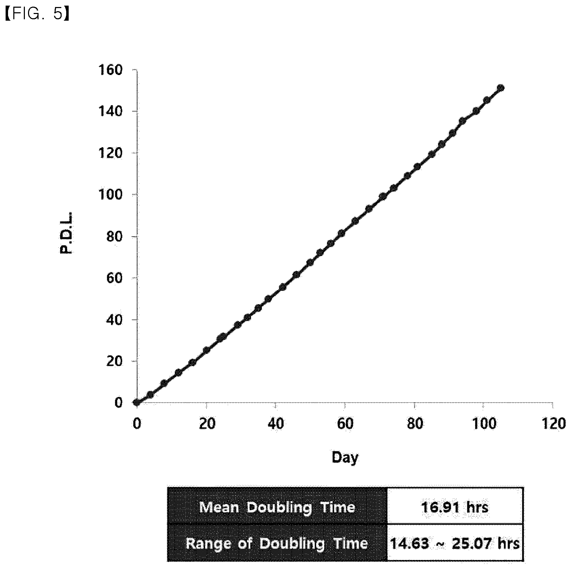

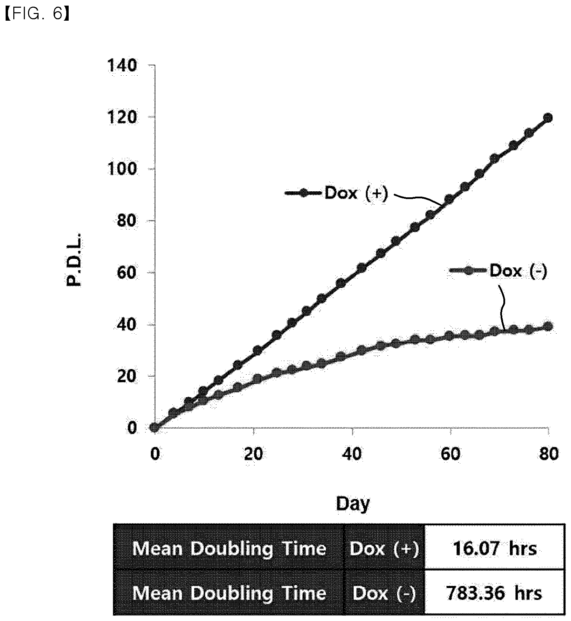

The proliferation rate of the BM102 cell line established in Example 3.1 was confirmed. 0.2×10⁶ to 0.8×10⁶ cells of the BM102 cell line were inoculated into a T175 flask and then cultured for 3 or 4 days with or without the addition of doxycycline, and during which, PDL (population doubling level) and cell viability were measured. PDL was calculated by the formula "PDL=X+3.222 (logY-logl)", where X denotes the initial PDL, I denotes the initial number of cells inoculated into the flask, and Y denotes the final number of cells. The proliferation rates of the established cell lines are shown in FIGS. 5 and 6.

As a result, as shown in FIG. 5, The BM102 cell line stably proliferated until 100 days or more. In addition, as shown in FIG. 6, it was confirmed that the cell proliferation rate of the BM102 cell line cultured without the addition of doxycycline for up to 80 days compared to that of the BM102 cell line cultured with the addition of doxycycline was gradually decreased.

### Experimental Example 3. Morphological confirmation of BM102 cell line according to subculture

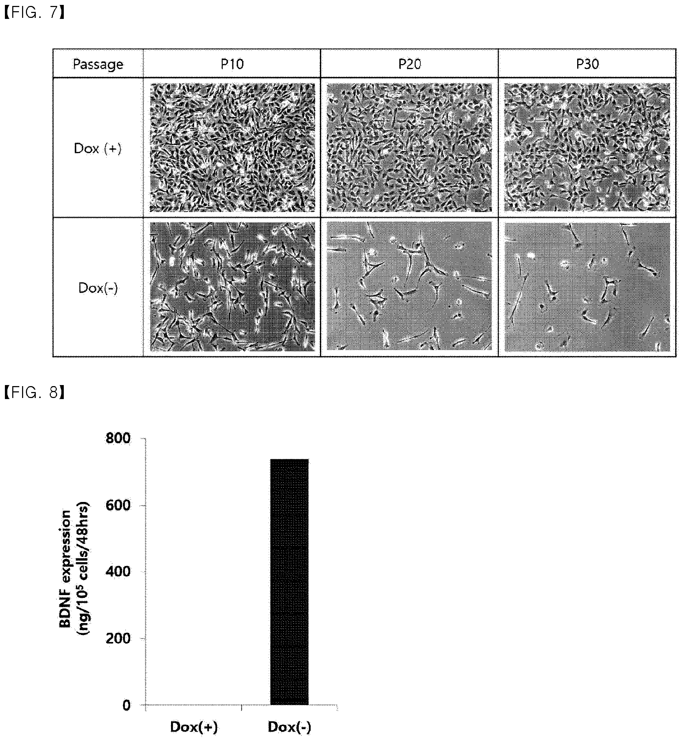

In order to confirm the morphological change according to the subculture of the BM102 cell line established in Example 3.1, in Experimental Example 2, the proliferation rate of the BM102 cell line was confirmed, and the cells were photographed using microscope, and the morphology of the BM102 cells is shown in FIG. 7.

As a result, as shown in FIG. 7, it was confirmed that BM102 cell line was not morphologically changed even after long-term culture for up to 100 days, and through this, it was found that a phenomenon such as cellular aging caused by long-term culture was not observed. However, when cultured without the addition of doxycycline, it was confirmed that the BM102 cell line was morphologically changed due to the expression of BDNF. This means that the expression of BDNF protein affects BM102 cells, thereby changing the cell characteristics.

### Experimental Example 4. Confirmation of expression of BDNF protein in BM102 cell line before irradiation

The expression of the BDNF protein in the BM102 cells established in Example 3.1 was confirmed by ELISA analysis. Specifically, the expression level of the BDNF protein was confirmed with the human BDNF DuoSet ELISA kit. The experiment was performed according to the manual included in each kit. The expression levels of the BDNF protein whose expression was induced for 48 hours from about 1×10⁵ cells in a medium with the addition or removal of doxycycline were shown in FIG. 8.

As a result, as shown in FIG. 8, about 0.2 ng of BDNF was detected in the medium with doxycycline, whereas about 740 ng of BDNF was detected in the medium without doxycycline.

**[Table 1]**

| Target protein | Expression level |
|---|---|
| BDNF | 740 ng/10⁵ cells |

As a result, as shown in Table 1, it was confirmed that about 740 ng/10⁵ cells of BDNF protein were expressed in the BM102 cell line before irradiation.

### Experimental Example 5. Confirmation of BDNF transgene in BM102 cell line

PCR was performed to confirm whether a gene was introduced into the BM102 cell line prepared in Example 3.1. Specifically, the BM102 cell line was transferred to a 15 ml tube containing 9 ml of PBS and then cell down was performed at 1,500 rpm for 5 minutes. After the PBS was completely removed, the pellet was suspended in 200 µl of PBS and then transferred to a 1.5ml tube. Thereafter, gDNA was prepared using NucleoSpin^{®} Tissue (MN, 740952.250), a mixture was prepared as shown in Table 2 below, and then PCR was performed with the steps shown in the Table 3 below. In this case, 100 ng of BM102 plasmid DNA was added as a positive control, and 1 µl of purified water was added as a negative control. The BM102 plasmid DNA can be separated and purified according to the method described in Korean Patent Application Laid-Open No. 2017-0093748.

**[Table 2]**

| | |
|---|---|
| Forward primer (SEQ ID NO: 10) (10 pmol/µl, Cosmogenetech synthesis) | 1µl |
| Reverse primer (SEQ ID NO: 11) (10 pmol/µl, Cosmogenetech synthesis) | 1µl |
| Specimen (100 *ng*/*µl)* | 1µl |
| Purified water | 17 µl |
| Total volume | 20 µl |

**[Table 3]**

| Step | Temperature | Time | Number |
|---|---|---|---|
| 1 | 95°C | 5 minutes | 1 |
| 2 | 95°C | 30 seconds | 35 |
| | 62°C | 30 seconds | |
| | 72°C | 40 seconds | |
| 3 | 72°C | 7 minutes | 1 |
| 4 | 4°C | Unlimited | 1 |

A 1% (v/v) agarose gel was put into an electrophoresis kit. 10 µl of DNA Size Marker was loaded into the first well, and from the next well, each 10 µl was loaded in the order of a negative control, a positive control, and 3 BM102 specimens. Thereafter, electrophoresis was performed at 100 V for 20 minutes, a gel photograph was taken, and the results are shown in FIG. 9. As a result, as shown in FIG. 9, all 3 BM102 cell lines were confirmed to have PCR products of the same size (0.6 kb) as the positive control.

### Experimental Example 6. Confirmation of transcriptome changes in BM102 cell line

In the BM102 cell line prepared in Example 3.1, transcriptome sequencing analysis was performed to confirm the change in gene expression caused by BDNF expression depending on whether doxycycline was treated.

The sequencing analysis was commissioned to Macrogen to secure the results. The test was performed according to NovaSeq 6000 System User Guide Document #1000000019358 v02 (illumina) using NovaSeq 6000 S4 Reagent Kit (illumina), and analysis was performed through NovaSeq sequencer and 1000000019358 v02 software (illumina). Transcriptomes that change according to the removal of doxycycline in the BM102 cell line are listed in FIGS. 10a, 10b and 10c.

As a result, as shown in FIG. 10a, it was confirmed that according to the removal of doxycycline, in the BM102 cell line, the expression of ANGPTL4, ANGPT1, CEND1, NFASC, GRAP2 and TRIM6 genes known to be involved in angiogenesis, inflammation, neurogenesis and immune system processes was increased, and the expression of IL2RB, IL6, IL1 B and PTGS2 was decreased.

In addition, as shown in FIG. 10b, it was confirmed that according to the removal of doxycycline, in the BM102 cell line, the expression of HLF, ROR2 and ICAM1 genes known to be involved in cell differentiation, migration and proliferation was increased, and the expression of CEBPB, EGR2, BMP8A, BEX2, KLF4, TNFSF15, PTBP3 and IGFBP2 genes known to be involved in apoptosis was decreased.

Additionally, according to the removal of doxycycline, in the BM102 cell line, transcriptome changes of micro RNA genes were also confirmed, and specifically, it was confirmed that the expression of MiR4444-1, MiR4444-2 and MiR1244-1 was increased, and the expression of MiR1244-4, MiR319l and MiRLET7D was decreased (FIG. 10c).

### Experimental Example 7. Irradiation test for BM102 cell line

For the BM102 cell line, a irradiation test using gamma rays and X-rays was performed in the same manner as in Example 4, and gamma rays and X-rays were used as the types of radiation to be irradiated.

First, after primary irradiation with gamma rays at irradiation doses of 80, 100, 120, and 140 Gy, the absorbed dose, colony formation or not, cell number and titer of the BM102 cell line were confirmed, and after secondary irradiation with gamma rays at doses of 200 and 300 Gy, the absorbed dose, colony formation or not, cell number and titer were respectively confirmed in the same manner.

The results are shown in Table 4 below.

**[Table 4]**

| | Primary experiment | | | | Secondary experiment | | | |
|---|---|---|---|---|---|---|---|---|
| Irradiation dose | Absorbed dose | Colony | Titer | Suitable/unsui table | Absorbed dose | Colony | Titer | Suitable/unsui table |
| (Gy) | (Gy) | Formati on or not | ng/5x10⁵/4 8hrs | | (Gy) | Formati on or not | ng/5x10⁵/4 8hrs | |
| 0 Gy | - | - | 1259.8 | - | - | - | - | - |
| 80 Gy | 79.5 | O | 960.2 | unsuita ble | - | - | - | - |
| 90 Gy | - | - | - | - | - | - | - | - |
| 100 Gy | 99.6 | X | 826.6 | suitable | - | - | - | - |
| 110 Gy | - | - | - | - | - | - | - | - |
| 120 Gy | 121.2 | X | 963.3 | suitable | - | - | - | - |
| 130 Gy | - | - | - | - | - | - | - | - |
| 140 Gy | 137.6 | X | 849.9 | suitable | - | - | - | - |
| 200 Gy | - | - | - | - | 219.7 | X | 400 or more | suitable |
| 300 Gy | - | - | - | - | 322.2 | X | 400 or more | suitable |

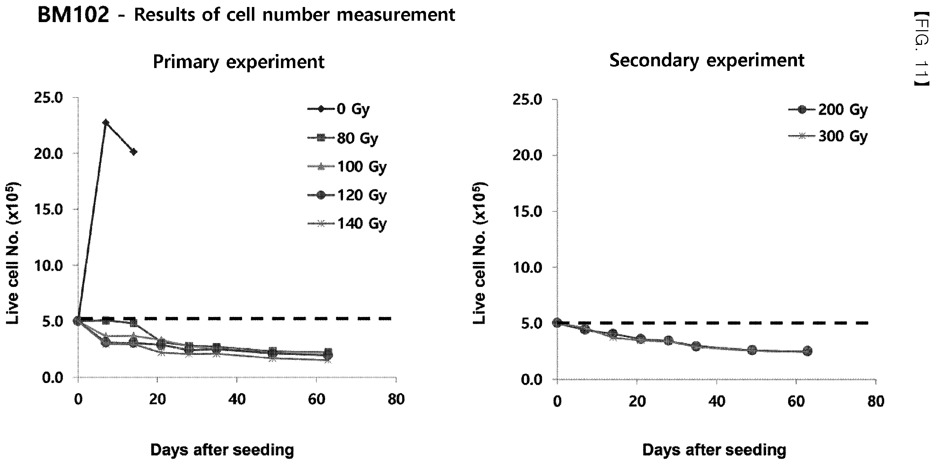

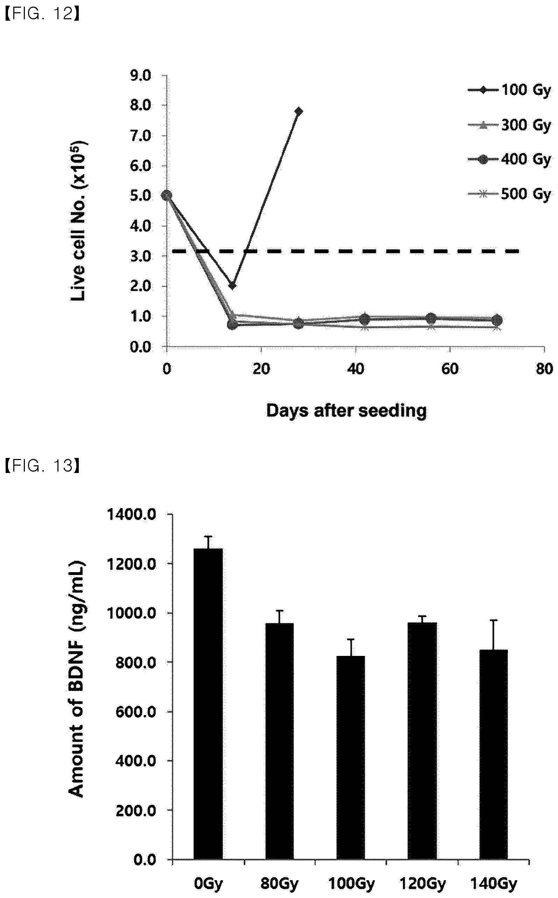

In addition, the absorbed dose, colony formation or not and titer were confirmed by first irradiation with irradiation doses of 100, 200, 300, 400, and 500 Gy, respectively, using X-rays, and the results are shown in Table 5 and FIG. 11, and second irradiation was performed with irradiation doses of 0, 50, 100, 200, 300, 400, and 500 Gy, respectively and the results are shown in Table 6 below and FIG. 12.

**[Table 5]**

| | | | | |
|---|---|---|---|---|
| | | | | |
| | | | | |

| | BM102 X-ray irradiation (primary) | | | |
|---|---|---|---|---|
| Irradiation dose | Absorbed dose | Colony | Titer | Suitable/unsuitable |
| (Gy) | (Gy) | Formation or not | ng/10⁵ | |
| 100 Gy | 62.5 | O | 122.4 | unsuitable |
| 300 Gy | 187.5 | X | 121.3 | suitable |
| 400 Gy | 250 | X | 120 | suitable |
| 500 Gy | 312.5 | X | 107.7 | suitable |

**[Table 6]**

| | BM102 X-ray irradiation (secondary) | | | | |
|---|---|---|---|---|---|
| Irradiation dose | Absorbed dose | After thawing | Colony | Titer | Suitable/unsuitable |
| (Gy) | (Gy) | Cell viability | Formation or not | ng/10⁵/60hr | |
| 0 Gy | - | 96.8 | NA | 325.1 | NA |
| 50 Gy | 41.3 | 96.7 | X | 247.5 | suitable |
| 100 Gy | 83.1 | 95.1 | X | 252.1 | suitable |
| 200 Gy | 155.5 | 95.6 | X | 249.4 | suitable |
| 300 Gy | 232.1 | 98.9 | X | 250.7 | suitable |
| 400 Gy | NA* | 96.4 | X | 240.7 | suitable |
| 500 Gy | NA* | 96.5 | X | 191.7 | suitable |

### Experimental Example 7.1. Colony formation or not of the irradiated BM102 cell line

For the BM102 cell line, to check whether colonies are formed after irradiating gamma rays and X-rays in the above ranges, respectively, the cells were aliquoted into 12 wells per vial so that there were 5×10⁵ cells in a well in a 6-well plate, and a total of two 6-well plates were aliquoted. The plate was shaken to spread the cells evenly, the medium was changed every 3 or 4 days in a 37°C, 5% CO₂ incubator, and it was visually observed whether colonies were formed using a microscope.

As a result, as shown in Table 4 above, upon irradiating gamma rays, colonies were formed when the irradiation dose was 80 Gy, and it was confirmed that colony formation was not made in a range higher than that. When colonies were formed, the radiation absorbed dose of the cell line was confirmed to be 79.5 Gy, and the maximum absorbed dose confirmed experimentally was about 322 Gy.

In addition, as a result of X-ray irradiation, it was also confirmed whether colonies were formed. As shown in Tables 5 and 6 above, in the primary experiment, when the irradiation dose was 100 Gy, colonies were formed, confirming that it was not suitable for use as a clinical sample, and colonies were not formed in a range beyond that. When colonies were formed, the absorbed dose of the cell line was found to be 62.5 Gy.

On the other hand, as confirmed in the secondary X-ray irradiation experiment, when irradiated with 100 Gy, the absorbed dose was measured to be 83.1 Gy. At this time, no colonies were formed. That is, even when irradiated with the same irradiation dose, there may be differences in absorbed dose, and it was confirmed that when the dose absorbed into the cells was 62.5Gy, it was not suitable for use as a clinical sample, but it can be used in the case of 83. 1Gy. Therefore, when radiation in a range exceeding about 80 Gy is absorbed based on the absorbed dose, it can be predicted that the cell line of the present invention can be used as a clinical sample.

### Experimental Example 7.2. Results of cell number measurement of the irradiated BM102 cell line

In order to test whether the colony was formed, a cell number measurement test was performed using the inoculated cells. For the BM102 cell line irradiated with gamma rays in Experimental Example 7, on each 7, 14, 28, 35, 49, and 63 days based on the cell thawing day, the attached cells were detached using trypsin, and then stained with trypan blue and the total number of cells and the number of viable cells were counted in a hematocytometer.

As a result, as shown in Table 7 below and FIG. 11, in both the primary irradiation test and the secondary irradiation test using gamma rays, it was confirmed that cells did not proliferate during irradiation when observed up to day 63.

**[Table 7]**

| Time | Item | Primary test (Dose) | | | | | Secondary test (Dose) | |
|---|---|---|---|---|---|---|---|---|
| | | 0 Gy | 80 Gy | 100 Gy | 120 Gy | 140 Gy | 200 Gy | 300 Gy |
| Day 0 | Number of cells (x10⁵) | 5.0 | 5.0 | 5.0 | 5.0 | 5.0 | 5.0 | 5.0 |
| | Viability (%) | 91.5 | 91.9 | 93.9 | 93.5 | 94.9 | 90.2 | 93.9 |
| Day 7 | Number of cells (x10⁵) | 22.75 | 5.09 | 3.91 | 3.19 | 2.95 | 4.39 | 4.55 |
| | Viability (%) | 93.1 | 93.9 | 97.7 | 98.0 | 97.6 | 90.5 | 91.6 |
| Day 14 | Number of cells (x10⁵) | 20.13 | 4.83 | 3.70 | 3.06 | 2.92 | 4.03 | 3.69 |
| | Viability (%) | 84.5 | 88.2 | 87.5 | 90.2 | 91.8 | 89.2 | 89.5 |
| Day 21 | Number of cells (x10⁵) | - | 3.13 | 3.32 | 2.88 | 2.21 | 3.57 | 3.45 |
| | Viability (%) | - | 81.3 | 84.3 | 83.6 | 74.1 | 89.9 | 88.5 |
| Day 28 | Number of cells (x10⁵) | - | 2.81 | 2.77 | 2.44 | 2.08 | 3.40 | 3.37 |
| | Viability (%) | - | 86.8 | 90.6 | 89.6 | 87.2 | 88.3 | 89.1 |
| Day 35 | Number of cells (x10⁵) | - | 2.69 | 2.62 | 2.53 | 2.11 | 2.93 | 2.88 |
| | Viability (%) | - | 85.6 | 84.3 | 87.5 | 85.9 | 85.4 | 84.3 |
| Day 49 | Number of cells (x10⁵) | - | 2.33 | 2.26 | 2.14 | 1.70 | 2.57 | 2.56 |
| | Viability (%) | - | 84.8 | 83.7 | 83.9 | 74.6 | 80.9 | 79.6 |
| Day 63 | Number of cells (x10⁵) | - | 2.25 | 2.23 | 1.98 | 1.55 | 2.48 | 2.47 |
| | Viability (%) | - | 83.2 | 82.1 | 81.5 | 71.6 | 79.4 | 78.6 |

In addition, for the cell lines subjected to irradiation experiment using X-rays, as a result of measuring the number of cells at each 14, 28, 42, 56, and 70 days after the cell line was thawed, as shown in Table 8 below and FIG. 12, it was confirmed that there were no additionally proliferating cells for all periods observed in each of 300, 400, and 500 Gy irradiation groups.

**[Table 8]**

| Time | Item | Dose | | | |
|---|---|---|---|---|---|
| | | 100 Gy | 300 Gy | 400 Gy | 500 Gy |
| Day 0 | Number of cells (x10⁵) | 5.0 | 5.0 | 5.0 | 5.0 |
| | Viability (%) | 92.5 | 93.8 | 94.3 | 95.5 |
| Day 14 | Number of cells (x10⁵) | 2.01 | 1.05 | 0.71 | 0.83 |
| | Viability (%) | 92.0 | 81.7 | 88.3 | 88.5 |
| Day 28 | Number of cells (x10⁵) | 7.79 | 0.85 | 0.74 | 0.73 |
| | Viability (%) | 79.9 | 71.2 | 63.4 | 66.9 |
| Day 42 | Number of cells (x10⁵) | - | 0.99 | 0.89 | 0.64 |
| | Viability (%) | - | 77.5 | 71.0 | 60.7 |
| Day 56 | Number of cells (x10⁵) | - | 0.96 | 0.94 | 0.66 |
| | Viability (%) | - | 74.0 | 76.0 | 69.7 |
| Day 70 | Number of cells (x10⁵) | - | 0.94 | 0.85 | 0.64 |
| | Viability (%) | - | 73.8 | 74.8 | 69.9 |

### Experimental Example 7.3. Confirmation of BDNF expression in the irradiated BM102 cell line

Some of the BM102 cells irradiated in Experimental Example 7 were aliquoted into a total of 1 well per vial so that 1×10⁵ or 5×10⁵ cells were placed in a well in a 12-well plate for titer test. The plate was shaken to spread the cells evenly, and it was cultured for 48 hours or 60 hours in a 37°C, 5% CO₂ incubator.

To measure the expression level of BDNF, a supernatant was obtained from the culture medium, centrifuged at 5,000 rpm at 4°C for 5 minutes, and the supernatant was divided into 4 e-tubes by 200 µL each and cryopreserved at - 80°C. The expression level of BDNF protein was analyzed using a BDNF assay kit (DBD00, R&D systems, USA).

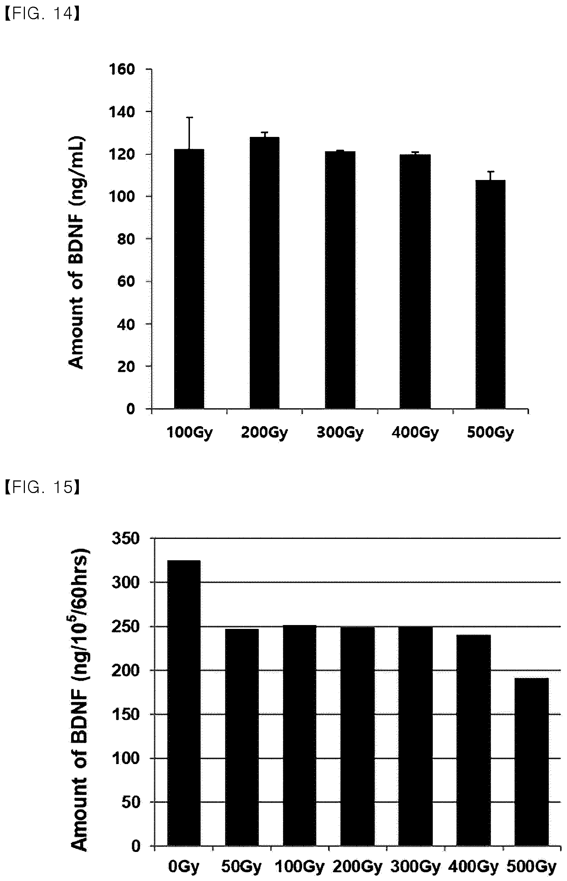

In the case of the BM102 cell line irradiated with gamma rays, as shown in Table 4 and FIG. 13, it was confirmed that the expression level of BDNF was maintained at an appropriate level (500 ng/5×10⁵/48hrs) even during irradiation, and in the case of X-ray irradiation, as confirmed in Table 5 and FIGS. 14 and 15, it was confirmed that the expression level of BDNF was maintained even at high irradiation dose (100 ng/10⁵/48hrs or more or 400 ng/10⁵/60hrs or more).

### Experimental Example 8. Irradiation test for BM01A cell line

In the same manner as in Experimental Example 7, a irradiation test was performed on the BM01A cell line. Gamma rays were used as the type of radiation to be irradiated, and after primary irradiation with gamma rays at irradiation doses of 90, 100, 110, and 120 Gy, the absorbed dose, colony formation or not, cell number and titer were confirmed, and after secondary irradiation with gamma rays at doses of 140, 160 and 180 Gy, the absorbed dose, colony formation or not, cell number and titer were respectively confirmed in the same manner. The results are shown in Table 9 below.

**[Table 9]**

| | Primary experiment | | | | Secondary experiment | | | |
|---|---|---|---|---|---|---|---|---|
| Irradiati on dose | Absorbe d dose | Colony | Titer | Suitable/ unsuitabl e | Absorb ed dose | Colony | Titer | Suitable /unsuita ble |
| (Gy) | (Gy) | Formati on or not | (ng/5×1 0⁵/48hrs ) | | (Gy) | Formati on or not | (ng/5×1 0⁵/48hrs ) | |
| 0 Gy | - | - | 540.7 | - | - | - | 611.3 | - |
| 90 Gy | 95.6 | X | 1238.6 | suitable | - | - | - | - |
| 100 Gy | 108 | X | 896.1 | suitable | - | - | - | - |
| 110 Gy | 120.6 | X | 909 | suitable | - | - | - | - |
| 120 Gy | 131.1 | X | 1063.4 | suitable | - | - | - | - |
| 140 Gy | - | - | - | - | 154.1 | X | 1018.3 | suitable |
| 160 Gy | - | - | - | - | 173.8 | X | 870 | suitable |
| 180 Gy | - | - | - | - | 191.4 | X | 993.4 | suitable |

### Experimental Example 8.1. Colony formation or not of the irradiated BM01A cell line

The BM01A cell line was aliquoted in a 6-well plate at the number of 3×10⁵ cells/well, and after culturing in the same manner as in Experimental Example 7.1, colony formation or not was confirmed in the medium.

As a result, as shown in Table 9 above, colonies were not formed at all irradiation doses in the range of 90 to 120 Gy and 140 to 180 Gy, and it was confirmed that the minimum absorbed dose at which colonies were not formed was 95.6 Gy and the maximum absorbed dose was 191.4 Gy.

### Experimental Example 8.2. Results of cell number measurement of the irradiated BM01A cell line

In the same manner as in Experimental Example 7.2., a test for measuring the number of cells in the BM01A cell line irradiated with gamma rays was performed.

On 7, 14, 28, 35, and 42 days based on the cell thawing day, the attached cells were detached using trypsin, and then stained with trypan blue and the total number of cells and the number of viable cells were counted in a hematocytometer.

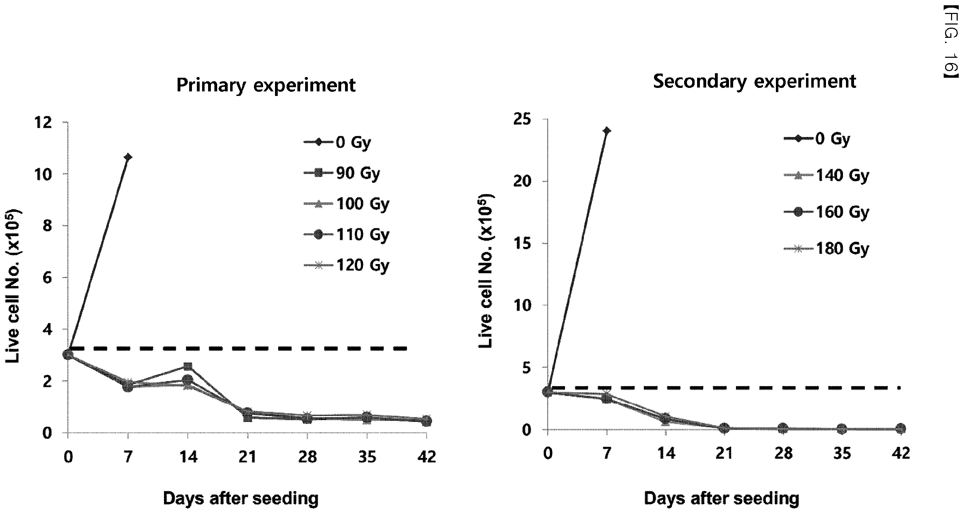

As a result, as shown in Table 10 below and FIG. 16, when the BM01A cell line was irradiated with gamma rays, it was confirmed that the number of cells did not proliferate when observed up to day 42.

**[Table 10]**

| Time | Item | Primary test (Dose) | | | | | Secondary test (Dose) | | | |
|---|---|---|---|---|---|---|---|---|---|---|
| | | 0 Gy | 90 Gy | 100 Gy | 110 Gy | 120 Gy | 0 Gy | 140 Gy | 160 Gy | 180 Gy |
| Day 0 | Number of cells (×10⁵) | 3.0 | 3.0 | 3.0 | 3.0 | 3.0 | 3.0 | 3.0 | 3.0 | 3.0 |
| | Viability (%) | 92.9 | 99.7 | 99.0 | 99.4 | 87.7 | 90.2 | 91.0 | 89.4 | 92.7 |
| Day 7 | Number of cells (×10⁵) | 9.44 | 1.66 | 1.57 | 1.32 | 1.70 | 24.03 | 2.44 | 2.42 | 2.86 |
| | Viability (%) | 88.8 | 89.5 | 88.5 | 75.0 | 87.1 | 86.1 | 71.7 | 73.0 | 75.2 |
| Day 14 | Number of cells (×10⁵) | - | 1.18 | 0.89 | 1.14 | 0.98 | - | 0.62 | 0.85 | 1.04 |
| | Viability (%) | - | 46.4 | 48.3 | 56.3 | 53.1 | - | 26.3 | 29.7 | 36.0 |
| Day 21 | Number of cells (×10⁵) | - | 0.23 | 0.30 | 0.22 | 0.23 | - | 0.14 | 0.08 | 0.11 |
| | Viability (%) | - | 39.7 | 38.6 | 29.2 | 29.0 | - | 3.6 | 2.5 | 3.5 |
| Day 28 | Number of cells (×10⁵) | - | 0.14 | 0.08 | 0.09 | 0.08 | - | 0.04 | 0.07 | 0.05 |
| | Viability (%) | - | 26.8 | 13.4 | 16.4 | 12.6 | - | 1.9 | 2.9 | 2.7 |
| Day 35 | Number of cells (×10⁵) | - | 0.04 | 0.06 | 0.03 | 0.04 | - | 0.03 | 0.03 | 0.02 |
| | Viability (%) | - | 6.3 | 12.0 | 4.9 | 5.9 | - | 4.0 | 3.7 | 2.3 |
| Day 42 | Number of cells (×10⁵) | - | 0.01 | 0.02 | 0.01 | 0.02 | - | 0.01 | 0.03 | 0.02 |
| | Viability (%) | - | 3.2 | 4.2 | 2.1 | 3.8 | - | 0.6 | 2.3 | 1.6 |

### Experimental Example 8.3. Confirmation of BDNF expression level in the irradiated BM01A cell line

For titer test of the BM01A cell line irradiated in Experimental Example 8, cells were cultured and cryopreserved in the same manner as in Experimental Example 7.3. The BDNF expression level of BM01A was analyzed using a BDNF assay kit (DY248, R&D systems, USA).

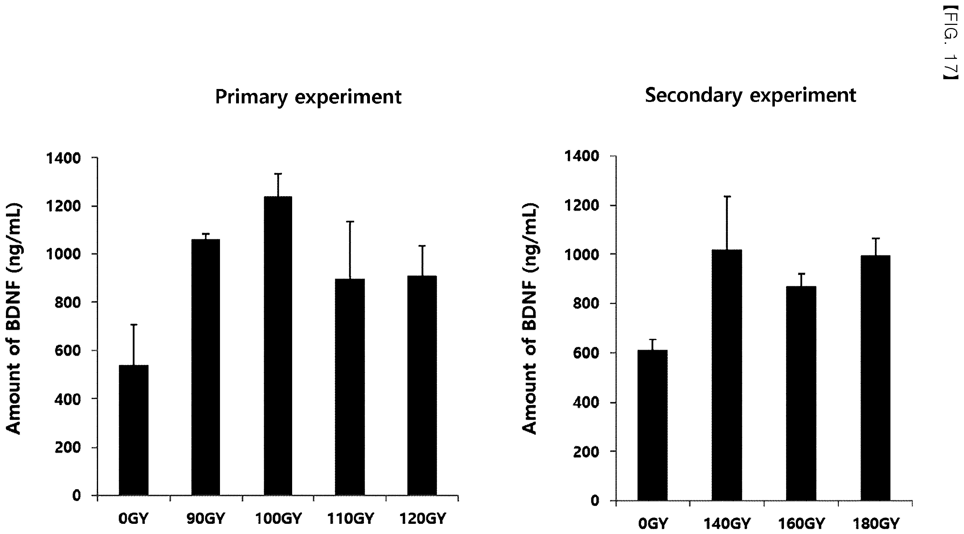

As a result, as shown in Table 9 and FIG. 17, upon irradiation with gamma rays, it was confirmed that the expression level of BDNF was maintained at a high level compared to the case where no irradiation was performed.

### Experimental Example 9. Irradiation test for BM03 cell line

For the BM03 cell line, which is an immortalized stem cell expressing TRAIL and CD, a irradiation test was performed in the same manner as in Experimental Example 7. The irradiation test of the BM03 strain utilized gamma rays, and the results were confirmed by irradiating low-level and high-level gamma rays, respectively.

For low-level gamma rays, BM03 (BM03-P005, 1×10⁷ cells/ml/vial) cryopreserved after filling in a vial was irradiated using a low-level irradiator at the Advanced Radiation Technology Institute of Korea Atomic Energy Research Institute, and irradiation doses of 0, 10, 20, 30, 40, 50, 60, 70 Gy were respectively irradiated and the absorbed dose, colony formation or not, cell number and titer were confirmed as shown in Table 11 below.

**[Table 11]**

| Low level | Primary experiment | | | | | | | | | | |
|---|---|---|---|---|---|---|---|---|---|---|---|
| Irradia tion dose | Absor bed dose* | Colony formati on or not | Titer (5×10^{(a+2)}) | | | | | | | | Suita ble/u nsuit able |
| | | | TRAIL (a) | | | | CD::UPRT (a) | | | | |
| (Gy) | (Gy) | | Day 0 | Day 2 | Day 7 | Day 14 | Day 0 | Day 2 | Day 7 | Day 14 | |
| 0 Gy | (0 Gy) | - | 8.92 | 9.26 | 8.82 | 9.20 | 8.64 | 9.17 | 8.66 | 9.08 | - |
| 10 Gy | 10 Gy | O | 8.62 | 9.34 | 8.76 | 8.98 | 8.38 | 9.20 | 8.61 | 8.81 | unsui table |
| 20 Gy | 20 Gy | O | 8.65 | 9.19 | 8.60 | 8.85 | 8.40 | 9.06 | 8.43 | 8.71 | unsui table |
| 30 Gy | 30 Gy | O | 9.13 | 9.13 | 8.81 | 8.45 | 8.73 | 9.01 | 8.71 | 8.45 | unsui table |
| 40 Gy | 40 Gy | O | 8.79 | 8.93 | 8.62 | 8.35 | 8.63 | 8.78 | 8.50 | 8.23 | unsui table |
| 50 Gy | 50 Gy | O | 8.60 | 8.71 | 8.76 | 8.46 | 8.40 | 8.70 | 8.68 | 8.41 | unsui table |
| 60 Gy | 60 Gy | X | 8.65 | 8.94 | 9.04 | 8.59 | 8.42 | 8.96 | 8.96 | 8.53 | unsui table |
| 70 Gy | 70 Gy | X | 9.06 | 9.12 | 8.94 | 8.58 | 8.75 | 9.13 | 8.78 | 8.56 | suita ble |

In addition, in the same manner, a high-level gamma-ray irradiation test was performed, and for high-level gamma rays, the primary test at doses of 0, 30, 40, 60, 70 Gy, and the secondary test at doses of 0, 100, 110, 120, 130Gy were performed to measure absorbed dose and colony formation or not. BM03 (1×10⁷ cells/ml/vial) cryopreserved after filling in a vial was irradiated using a high-level irradiator at the Advanced Radiation Technology Institute of Korea Atomic Energy Research Institute, and absorbed dose in each test was measured using an Alanine dosimeter. The results were confirmed as shown in Table 12 below.

**[Table 12]**

| High level | Primary experiment | | | Secondary experiment | | |
|---|---|---|---|---|---|---|
| Irradiatio n dose | Absorbed dose | Colony | Suitable/u nsuitable | Absorbed dose | Colony | Suitable/u nsuitable |
| (Gy) | (Gy) | Formation or not | | (Gy) | Formation or not | |
| 0 Gy | - | - | - | - | - | - |
| 30 Gy | 31.8 | O | unsuitable | - | - | - |
| 40 Gy | 40.4 | O | unsuitable | - | - | - |
| 50 Gy | 52.2 | O | unsuitable | - | - | - |
| 60 Gy | 61.2 | O | unsuitable | - | - | - |
| 100 Gy | - | - | - | 100 | X | suitable |
| 110 Gy | - | - | - | 110 | X | suitable |
| 120 Gy | - | - | - | 120 | X | suitable |
| 130 Gy | - | - | - | 130 | X | suitable |

### Experimental Example 9.1. Colony formation or not of the irradiated BM03 cell line

In order to confirm whether colonies were formed on the BM03 cells of Experimental Example 9, it was confirmed whether colony formation of the cell line was made in the same manner as in Experimental Example 7.1. In Experimental Example 9, as a result of confirming whether colonies were formed in BM03 irradiated with low-level gamma rays and BM03 irradiated with high-level gamma rays, as shown in Tables 11 and 12 above, when irradiated with low-level gamma rays, colonies were formed, especially at an irradiation dose of 50 Gy or less, and when irradiation with high-level gamma rays, colonies were formed at an irradiation dose of 60 Gy or less, confirming that it was not suitable for use as a clinical sample. However, it was confirmed that colonies were not formed at 70 Gy or more for low-level gamma rays and 100 Gy or more for high-level gamma rays.

### Experimental Example 9.2. Cell number measurement of the irradiated BM03 cell line

With respect to the cells cultured in Experimental Example 9.1, in the case of the BM03 cell line irradiated with low-level gamma rays, on days 2, 7, 14, 28, 35, 42, and 49 from the cell thawing day, it was confirmed whether the number of cultured cells increased by counting the number of the attached cells.

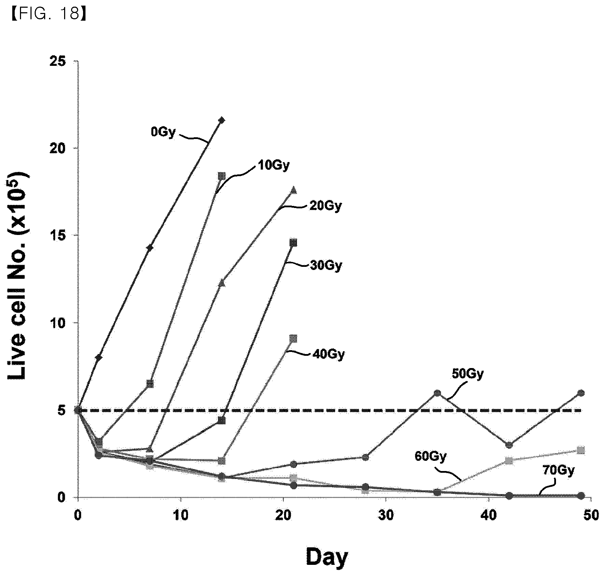

As a result, as shown in Table 13 below and FIG. 18, in the 10 Gy irradiation group, the number of cells greater than the inoculated number was confirmed from Day 7, and in the 20 Gy irradiation group on Day 14 and 30, in the 40 Gy irradiation group on Day 21, and in the 50 Gy irradiation group on 35, the number of cells greater than the inoculated number was confirmed. In addition, in the 60 Gy irradiation group, a smaller number of cells than the inoculated number was maintained, but the number of cells increased from Day 42, and the number of viable cells was continuously decreased at 70 Gy. This shows that when the irradiation dose and absorbed dose of low-level gamma rays is 60 Gy or less, there is a possibility of cell proliferation.

**[Table 13]**

| Time | Item | Dose | | | | | | | |
|---|---|---|---|---|---|---|---|---|---|
| | | 0 Gy | 10 Gy | 20 Gy | 30 Gy | 40 Gy | 50 Gy | 60 Gy | 70 Gy |
| Day 0 (inocula tion) | Number of viable cells (×10⁵) | 5.0 | 5.0 | 5.0 | 5.0 | 5.0 | 5.0 | 5.0 | 5.0 |
| | Viability (%) | 89.9 | 89.1 | 93.6 | 92.0 | 93.6 | 92.0 | 90.8 | 90.6 |
| Day 2 | Number of viable cells (×10⁵) | 8.0 | 3.2 | 2.6 | 2.6 | 2.8 | 2.6 | 2.6 | 2.4 |
| | Viability (%) | 84.3 | 70.0 | 52.5 | 60.8 | 55.5 | 56.6 | 47.2 | 49.8 |
| Day 7 | Number of viable cells (×10⁵) | 14.3 | 6.5 | 2.8 | 2.0 | 2.2 | 1.9 | 1.8 | 2.1 |
| | Viability (%) | 93.1 | 91.2 | 75.8 | 79.8 | 81.7 | 86.2 | 86.3 | 78.0 |
| Day 14 | Number of viable cells (×10⁵) | 21.6 | 18.4 | 12.3 | 4.4 | 2.1 | 1.1 | 1.1 | 1.1 |
| | Viability (%) | 92.0 | 94.4 | 90.5 | 78.6 | 73.8 | 59.2 | 63.2 | 70.8 |
| Day 21 | Number of viable cells (×10⁵) | 10.4 | 18.4 | 17.6 | 14.6 | 9.1 | 1.9 | 1.1 | 0.7 |
| | Viability (%) | 86.7 | 91.8 | 92.9 | 86.1 | 86.9 | 67.4 | 62.3 | 69.8 |
| Day 28 | Number of viable cells (×10⁵) | - | - | - | 17.3 | 8.1 | 2.3 | 0.4 | 0.6 |
| | Viability (%) | - | - | - | 93.8 | 86.0 | 88.7 | 74.6 | 73.0 |
| Day 35 | Number of viable cells (×10⁵) | - | - | - | - | - | 6.0 | 0.3 | 0.3 |
| | Viability (%) | - | - | - | - | - | 79.8 | 61.3 | 47.9 |
| Day 42 | Number of viable cells (×10⁵) | - | - | - | - | - | 3.0 | 2.1 | 0.1 |
| | Viability (%) | - | - | - | - | - | 35.4 | 66.9 | 68.1 |
| Day 49 | Number of | - | - | - | - | - | 6.0 | 2.7 | 0.1 |
| | viable cells (×10⁵) | | | | | | | | |
| | Viability (%) | - | - | - | - | - | 68.9 | 49.3 | 16.3 |

In addition, for the BM03 cell line irradiated with high-level gamma rays in the same manner, the number of attached cells was counted on days 7, 14, 21, 28 (primary), and 7, 14, 21, 28, 35, 42, 49, 63 (secondary) from the day of cell thawing.

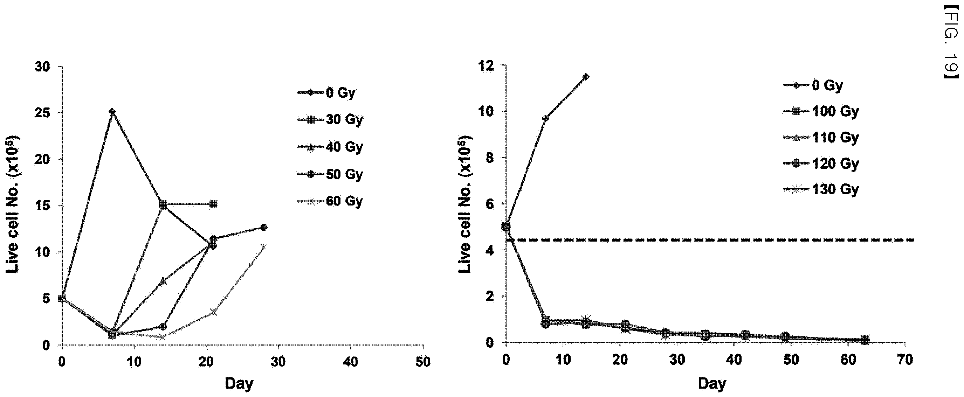

As a result, as shown in Table 14 below and FIG. 19, in the primary experiment, in the 30 Gy, 40 Gy irradiation groups, the number of cells greater than the inoculated number was confirmed from Day 14, and in the 50 Gy irradiation group on Day 21, and in the 60 Gy irradiation group on Day 28 the number of cells greater than the inoculated number was confirmed, but as a result of the secondary experiment, it was confirmed that there were no proliferating cells when observed up to day 63 in the 100, 110, 120, and 130 Gy irradiation groups.

From these results, it can be seen that in the case of the BM03 cell line, the minimum absorbed dose should be 60 Gy or more.

**[Table 14]**

| TIME | ITEM | Dose (primary experiment) | | | | | Dose (secondary experiment) | | | | |
|---|---|---|---|---|---|---|---|---|---|---|---|
| | | 0 Gy | 30 Gy | 40 Gy | 50 Gy | 60 Gy | 0 Gy | 100 Gy | 110 Gy | 120 Gy | 130 Gy |
| Day 0 | Number of cells (×10⁵) | 5.0 | 5.0 | 5.0 | 5.0 | 5.0 | 5.0 | 5.0 | 5.0 | 5.0 | 5.0 |
| | Viability (%) | 84.2 3 | 86.18 | 86.5 8 | 87.3 3 | 92.52 | 82.0 | 72.5 | 85.4 | 84.5 | 85.2 |
| Day 7 | Number of | 25.0 | 1.43 | 1.08 | 1.01 | 1.42 | 9.69 | 0.98 | 0.98 | 0.80 | 0.93 |
| | cells (×10⁵) | 8 | | | | | | | | | |
| | Viability (%) | 93.7 5 | 83.64 | 80.1 7 | 85.4 1 | 97.92 | 90.9 | 71.8 | 66.1 | 75.7 | 77.1 |
| Day 14 | Number of cells (×10⁵) | 14.9 2 | 15.17 | 6.91 | 1.97 | 0.83 | 11.5 0 | 0.76 | 0.80 | 0.84 | 0.96 |
| | Viability (%) | 89.8 | 88.0 | 88.3 | 79.8 | 67.0 | 93.7 | 72.5 | 77.8 | 68.9 | 71.0 |
| Day 21 | Number of cells (×10⁵) | 10.5 8 | 15.17 | 11.0 8 | 11.4 2 | 3.50 | - | 0.78 | 0.62 | 0.61 | 0.58 |
| | Viability (%) | 88.2 | 90.6 | 85.7 | 87.0 | 83.9 | - | 77.8 | 77.4 | 74.1 | 67.1 |
| Day 28 | Number of cells (×10⁵) | - | - | - | 12.6 7 | 10.50 | - | 0.42 | 0.46 | 0.37 | 0.32 |
| | Viability (%) | - | - | - | 95.0 | 90.0 | - | 57.2 | 69.0 | 56.7 | 68.1 |
| Day 35 | Number of cells (×10⁵) | | | | | | - | 0.39 | 0.23 | 0.27 | 0.28 |
| | Viability (%) | | | | | | - | 79.7 | 61.0 | 68.0 | 58.3 |
| Day 42 | Number of cells (×10⁵) | | | | | | - | 0.33 | 0.36 | 0.31 | 0.25 |
| | Viability (%) | | | | | | - | 63.7 | 71.4 | 79.7 | 66.8 |
| Day 49 | Number of cells (×10⁵) | | | | | | - | 0.25 | 0.15 | 0.23 | 0.16 |
| | Viability (%) | | | | | | - | 59.6 | 61.8 | 50.5 | 59.4 |
| Day 63 | Number of cells (×10⁵) | | | | | | - | 0.08 | 0.07 | 0.09 | 0.13 |
| | Viability (%) | | | | | | - | 44.3 | 41.7 | 60.5 | 62.3 |

### Experimental Example 9.3. Confirmation of gene expression level in the irradiated BM03 cell line

In order to measure the change in the cell titer after irradiation, after selecting a primer capable of confirming the inserted therapeutic genes TRAIL and CD::UPRT, the expression of the inserted gene was quantitatively measured through RT-qPCR reaction.

After harvesting cells immediately after thawing, and harvesting cells cultured for 2 days, 7 days and 14 days, respectively, RNA was extracted using Trizol, and cDNA was synthesized from 1 ug of RNA using 5X Prime Script RT Master Mix (Takara), and then RT-qPCR was performed using primers capable of specifically binding to each of TRAIL and CD.

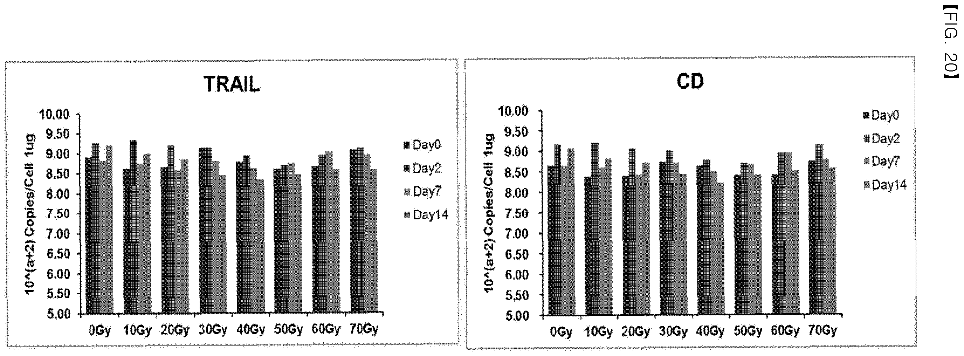

As a result, as shown in FIG. 20, no significant difference was found in the expression levels of the therapeutic genes TRAIL and CD expressed in the BM03 cell line irradiated with low-level gamma rays. It was confirmed that even when irradiated with high-level gamma rays, when viable cells were present, there was little change in titer according to the irradiation dose.

### Experimental Example 10. In vitro confirmation of tumorigenicity of BM102 cell line

The presence or absence of colony formation by anchorage-independent growth was confirmed in bone marrow-derived MSC, BM102 cell line, positive control (HeLa) and negative control (NIH3T3) in Soft Agar Gels using CytoSelect^{™} 96-well Cell Transformation Assay, and the presence or absence of tumor formation by cell transformation was quantified by measuring the absorbance by staining with MTS solution.

As a result, as shown in FIG. 21, colonies were formed by cell transformation in the positive control, and the negative control, bone marrow-derived MSC and BM102 cell line did not form colonies, and from this, it was confirmed that the BM102 cell line had no transforming ability and no *in vitro* tumorigenicity during cell culture.

### Experimental Example 11. In vitro confirmation of nerve cell protective and therapeutic effects of BM102 cell line

In order to confirm the nerve cell protective and therapeutic effects of the BM102 cell line, after co-culturing C6 glial cells, known to grow well by BDNF, with the BM102 cell line, an increase in the proliferation rate of C6 cells was confirmed in a cell number-dependent manner.

Specifically, C6 cells and BM102 cell line were co-cultured using a trans-well. After inoculating the same 2×10³ C6 cells in a 96-well plate, the BM102 cell line was diluted by 1/2 from 1×10⁴ cells and was inoculated into the trans-well to vary the number of co-cultured cells. After 24 hours of co-culture, the proliferation rate of C6 cells by the BDNF protein expressed in the BM102 cell line was quantified by treating the C6 cell medium in culture with MTS solution and measuring the absorbance.

As a result, as shown in FIG. 22, it was confirmed that the proliferation rate of C6 cells was increased, even without direct contact, by the amount of BDNF secretion, which increased in proportion to the number of BM102 cell lines.

From the above results, it was confirmed that the BM102 cell line can stably mass-produce BDNF enough to be used as a cell therapeutic agent, and that the BM102 cell line stably expressing BDNF can be used as a cell therapeutic agent for protecting or treating nerve cells.

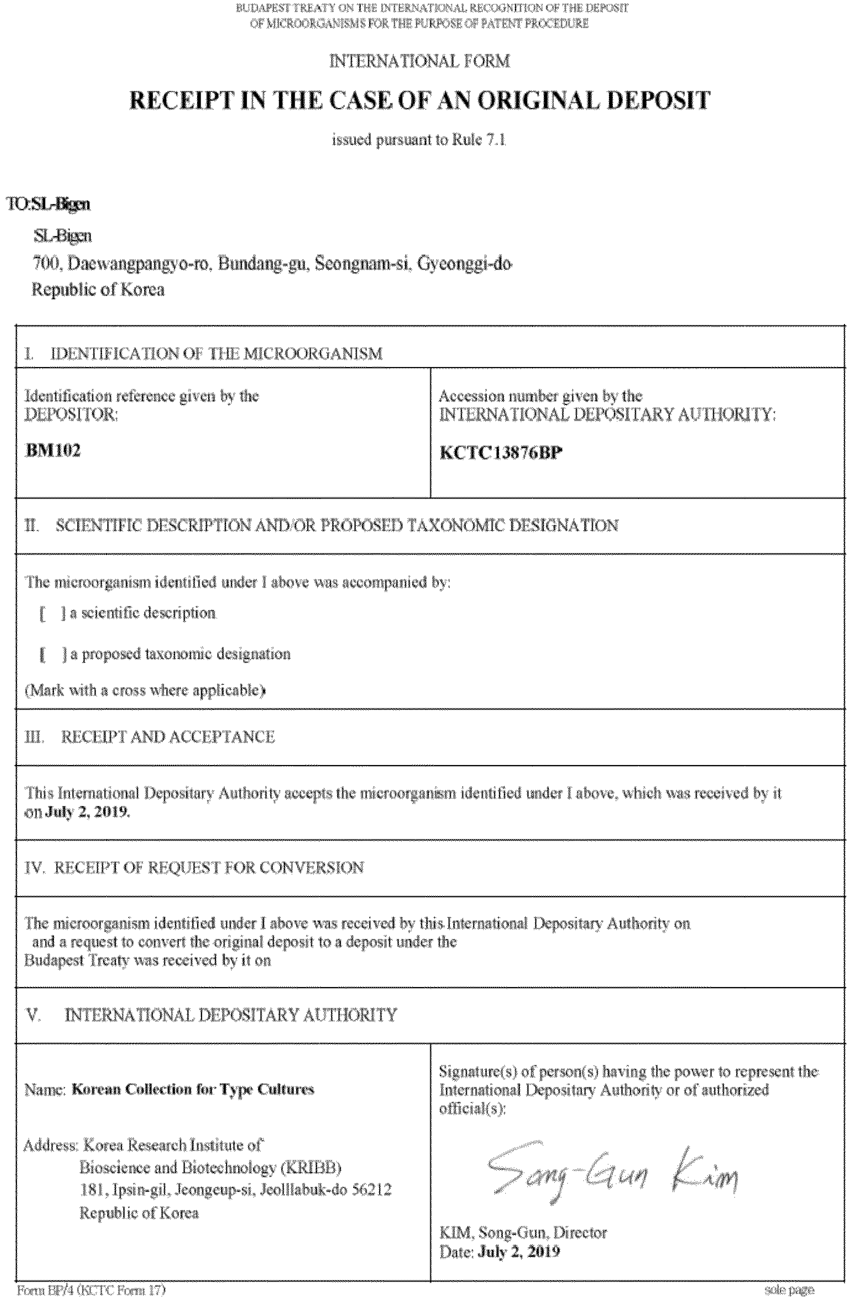
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO:SL-Bigen
SL-Bigen
700, Daewangpangyo-ro, Bundang-gu, Seongnam-si, Gyeonggi-do
Republic of Korea

I. IDENTIFICATION OF THE MICROORGANISM
Identification reference given by the
DEPOSITOR:
BM102 | Accession number given by the
INTERNATIONAL DEPOSITARY AUTHORITY:
KCTC13876BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on July 2, 2019.
IV. RECEIPT OF REQUEST FOR CONVERSION
The microorganism identified under I above was received by this International Depositary Authority on
and a request to convert the original deposit to a deposit under the
Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: July 2, 2019
Form BP/4 (KCTC Form 17) sole page

<110>    SLBIGEN Inc.

<120>    Method for producing immortalized stem cell and use thereof}

<130>    1067684

<150>    KR 10-2019-0089897
<151>    2019-07-24

<160>    13

<170>    KoPatentIn 3.0

<210>    1
<211>    255
<212>    PRT
<213>    Artificial Sequence

<220>
<223>    amino acid sequence of BDNF


<400>    1
Met Phe His Gln Val Arg Arg Val Met Thr Ile Leu Phe Leu Thr Met
  1               5                  10                  15

Val Ile Ser Tyr Phe Gly Cys Met Lys Ala Ala Pro Met Lys Glu Ala
             20                  25                  30

Asn Ile Arg Gly Gln Gly Gly Leu Ala Tyr Pro Gly Val Arg Thr His
         35                  40                  45

Gly Thr Leu Glu Ser Val Asn Gly Pro Lys Ala Gly Ser Arg Gly Leu
     50                  55                  60

Thr Ser Leu Ala Asp Thr Phe Glu His Val Ile Glu Glu Leu Leu Asp
 65                  70                  75                  80

Glu Asp Gln Lys Val Arg Pro Asn Glu Glu Asn Asn Lys Asp Ala Asp
                 85                  90                  95

Leu Tyr Thr Ser Arg Val Met Leu Ser Ser Gln Val Pro Leu Glu Pro
            100                 105                 110

Pro Leu Leu Phe Leu Leu Glu Glu Tyr Lys Asn Tyr Leu Asp Ala Ala
        115                 120                 125

Asn Met Ser Met Arg Val Arg Arg His Ser Asp Pro Ala Arg Arg Gly
    130                 135                 140

Glu Leu Ser Val Cys Asp Ser Ile Ser Glu Trp Val Thr Ala Ala Asp
145                 150                 155                 160

Lys Lys Thr Ala Val Asp Met Ser Gly Gly Thr Val Thr Val Leu Glu
                165                 170                 175

Lys Val Pro Val Ser Lys Gly Gln Leu Lys Gln Tyr Phe Tyr Glu Thr
            180                 185                 190

Lys Cys Asn Pro Met Gly Tyr Thr Lys Glu Gly Cys Arg Gly Ile Asp
        195                 200                 205

Lys Arg His Trp Asn Ser Gln Cys Arg Thr Thr Gln Ser Tyr Val Arg

210                 215                 220

Ala Leu Thr Met Asp Ser Lys Lys Arg Ile Gly Trp Arg Phe Ile Arg
225                 230                 235                 240

Ile Asp Thr Ser Cys Val Cys Thr Leu Thr Ile Lys Arg Gly Arg
                245                 250                 255

<210>    2
<211>    768
<212>    DNA
<213>    Artificial Sequence

<220>
<223>    nucleotide sequence of BDNF

<400>    2
atgttccacc aggtgagaag agtgatgacc atcctgttcc tgaccatggt gatcagctac      60

ttcggctgca tgaaggctgc ccctatgaag gaggccaaca tcagaggaca gggcggcctg     120

gcctaccccg gcgtgagaac ccacggcacc ctggagagcg tgaacggccc caaggccggc     180

agcagaggcc tgaccagcct ggccgacacc ttcgagcacg tgatcgagga gctgctggac     240

gaggaccaga aggtgagacc caacgaggag aacaacaagg acgccgacct gtacaccagc     300

agagtgatgc tgagcagcca ggtgcccctg gagcctcccc tgctgttcct gctggaggag     360

tacaagaact acctggacgc cgccaacatg agcatgagag tgagaagaca cagcgacccc     420

gccagaagag gcgagctgag cgtgtgcgac agcatcagcg agtgggtgac cgccgccgac     480

aagaagaccg ccgtggacat gagcggcggc accgtgaccg tgctggagaa ggtgcccgtg     540

agcaagggcc agctgaagca gtacttctac gagaccaagt gcaaccccat gggctacacc     600

aaggagggct gcagaggcat cgacaagaga cactggaaca gccagtgcag aaccacacag     660

agctacgtga gagccctgac catggacagc aagaagagaa tcggctggag attcatcaga     720

atcgacacca gctgcgtgtg caccctgacc atcaagagag gcagataa                  768

<210>    3
<211>    281
<212>    PRT
<213>    Artificial Sequence

<220>
<223>    Amino acid sequence of TRAIL

<400>    3


<210>   4
<211>   846
<212>   DNA
<213>   Artificial Sequence

<220>
<223>   nucleotide sequence of TRAIL


<400>   4
atggcaatga tggaggtcca gggggggcct tcactgggac agacatgcgt gctgatcgtc      60

atctttaccg tgctgctgca gtcactgtgc gtggctgtca catacgtgta tttcactaac     120

gagctgaagc agatgcagga caagtactca aaaagcggaa tcgcatgctt tctgaaagaa     180

gacgatagct attgggaccc taacgatgag gaatccatga actccccatg ttggcaggtg 240

aagtggcagc tgcgacagct ggtccggaaa atgatcctga ggactagtga ggaaactatt 300

tcaaccgtgc aggagaagca gcagaatatc agcccactgg tgcgggaaag aggaccacag 360

cgagtcgcag ctcacattac cggaacaagg ggccgcagca acaccctgag ctccccaaac 420

tccaagaatg agaaagccct gggcagaaag atcaattcct gggaatctag taggagtggg 480

cactcattcc tgagcaacct gcatctgcgc aatggggagc tggtcatcca tgaaaaaggc 540

ttctactata tctactctca gacctatttc cgatttcagg aggaaattaa ggagaacaca 600

aagaatgaca aacagatggt ccagtacatc tataaataca catcttaccc cgatcctatt 660

ctgctgatga agagtgcacg gaactcctgt tggtctaaag acgccgagta tgggctgtac 720

agcatctatc agggcgggat tttcgagctg aaggaaaatg atagaatctt tgtgagcgtc 780

accaacgagc atctgattga catggatcac gaggcttcat ttttcggggc attcctggtc 840

ggataa 846

<210> 5
<211> 373
<212> PRT
<213> Artificial Sequence

<220>
<223> amino acid sequence of CD

<400> 5
Met Val Thr Gly Gly Met Ala Ser Lys Trp Asp Gln Lys Gly Met Asp
1               5                   10                  15

Ile Ala Tyr Glu Glu Ala Ala Leu Gly Tyr Lys Glu Gly Gly Val Pro
            20                  25                  30

Ile Gly Gly Cys Leu Ile Asn Asn Lys Asp Gly Ser Val Leu Gly Arg
        35                  40                  45

Gly His Asn Met Arg Phe Gln Lys Gly Ser Ala Thr Leu His Gly Glu
    50                  55                  60

Ile Ser Thr Leu Glu Asn Cys Gly Arg Leu Glu Gly Lys Val Tyr Lys
65                  70                  75                  80

Asp Thr Thr Leu Tyr Thr Thr Leu Ser Pro Cys Asp Met Cys Thr Gly
                85                  90                  95

Ala Ile Ile Met Tyr Gly Ile Pro Arg Cys Val Val Gly Glu Asn Val
            100                 105                 110

Asn Phe Lys Ser Lys Gly Glu Lys Tyr Leu Gln Thr Arg Gly His Glu
        115                 120                 125

Val Val Val Val Asp Asp Glu Arg Cys Lys Lys Ile Met Lys Gln Phe
    130                 135                 140

Ile Asp Glu Arg Pro Gln Asp Trp Phe Glu Asp Ile Gly Glu Ser Ser
145                 150                 155                 160

Glu Pro Phe Lys Asn Val Tyr Leu Leu Pro Gln Thr Asn Gln Leu Leu
                165                 170                 175

Gly Leu Tyr Thr Ile Ile Arg Asn Lys Asn Thr Thr Arg Pro Asp Phe
            180                 185                 190

Ile Phe Tyr Ser Asp Arg Ile Ile Arg Leu Leu Val Glu Glu Gly Leu
        195                 200                 205

Asn His Leu Pro Val Gln Lys Gln Ile Val Glu Thr Asp Thr Asn Glu
    210                 215                 220

Asn Phe Glu Gly Val Ser Phe Met Gly Lys Ile Cys Gly Val Ser Ile
225                 230                 235                 240

Val Arg Ala Gly Glu Ser Met Glu Gln Gly Leu Arg Asp Cys Cys Arg
                245                 250                 255

Ser Val Arg Ile Gly Lys Ile Leu Ile Gln Arg Asp Glu Glu Thr Ala
            260                 265                 270

Leu Pro Lys Leu Phe Tyr Glu Lys Leu Pro Glu Asp Ile Ser Glu Arg
        275                 280                 285

Tyr Val Phe Leu Leu Asp Pro Met Leu Ala Thr Gly Gly Ser Ala Ile
    290                 295                 300

Met Ala Thr Glu Val Leu Ile Lys Arg Gly Val Lys Pro Glu Arg Ile
305                 310                 315                 320

Tyr Phe Leu Asn Leu Ile Cys Ser Lys Glu Gly Ile Glu Lys Tyr His
                325                 330                 335

Ala Ala Phe Pro Glu Val Arg Ile Val Thr Gly Ala Leu Asp Arg Gly
            340                 345                 350

Leu Asp Glu Asn Lys Tyr Leu Val Pro Gly Leu Gly Asp Phe Gly Asp
        355                 360                 365

Arg Tyr Tyr Cys Val
    370


<210>    6
<211>    1122
<212>    DNA
<213>    Artificial Sequence

<220>
<223>    nucleotide sequence of CD


<400>    6
atggtgaccg gcggcatggc cagcaagtgg gaccagaagg gcatggacat cgcctacgag        60

gaggccgctc tgggctacaa ggagggcggc gtgcccatcg gcggatgcct gatcaacaac       120

aaggacggca gcgtgctggg cagaggccac aacatgagat tccagaaggg cagcgccacc       180

ctgcacggcg agatcagcac cctggagaac tgcggcagac tggagggcaa ggtgtacaag       240

gacacaaccc tgtacaccac actgagcccc tgcgacatgt gcaccggcgc catcatcatg 300

tacggcatcc ccagatgcgt ggtgggcgag aacgtgaact tcaagagcaa gggcgagaag 360

tacctgcaga ccagaggcca cgaggtggtc gtcgtggacg acgagagatg caagaagatc 420

atgaagcagt tcatcgacga gagaccccag gactggttcg aggacatcgg cgagagcagc 480

gagcccttca agaacgtgta cctgctgccc cagaccaacc agctgctggg cctgtacacc 540

atcatcagaa acaagaacac caccagaccc gacttcatct tctacagcga cagaatcatc 600

agactgctgg tggaggaggg cctgaaccac ctgcccgtgc agaagcagat cgtggagacc 660

gacaccaacg agaacttcga gggcgtgagc ttcatgggca aaatctgcgg cgtgagcatc 720

gtgagagccg gcgagagcat ggagcagggc ctgagagact gctgcagaag cgtgagaatc 780

ggcaagatcc tgatccagag agacgaggag accgccctgc ccaagctgtt ctacgagaag 840

ctgcccgagg acatcagcga gagatacgtg ttcctgctgg accccatgct ggccaccggc 900

ggcagcgcca tcatggccac cgaggtgctg atcaagagag gcgtgaagcc cgagagaatc 960

tacttcctga acctgatctg cagcaaggag ggcatcgaga agtaccacgc tgccttcccc 1020

gaggtgagaa tcgtgaccgg cgccctggac agaggcctgg acgagaacaa gtacctggtg 1080

cccggcctgg gcgacttcgg cgacagatac tactgcgtgt aa 1122

<210> 7
<211> 1365
<212> DNA
<213> Artificial Sequence

<220>
<223> nucleotide sequence of c-Myc

<400> 7
atggatttct ttcgcgtcgt cgagaaccag cagccacccg ccactatgcc tctgaacgtg 60

tcttttacta acaggaacta tgatctggat tacgacagcg tgcagcccta cttctattgc 120

gatgaggaag agaactttta tcagcagcag cagcagagcg agctgcagcc acctgcacct 180

tccgaagaca tttggaagaa attcgagctg ctgcctacac cacccctgtc tccaagtcgg 240

agaagcggcc tgtgttcacc cagctacgtg gccgtcactc ctttcagcct gaggggggac 300

aatgatggcg ggggaggctc cttttctaca gccgatcagc tggaaatggt gactgagctg 360

ctggggggag acatggtcaa ccagagcttc atttgcgatc ctgacgatga aacttttatc 420

aagaacatca tcatccagga ctgtatgtgg tcaggcttta gcgccgctgc aaagctggtg 480

tctgagaaac tggcaagtta tcaggccgct cggaaagata gtgggtcacc taacccagct 540

agaggacact ccgtgtgctc tacaagctcc ctgtacctgc aggacctgag cgcagccgct 600

tccgagtgta ttgatccctc cgtggtcttc ccctatcctc tgaatgactc tagttcaccc 660

aagagttgcg catcacagga cagctccgcc ttttcacctt ctagtgatag cctgctgtca 720

agcactgaat cctctccaca gggcagccca gagccactgg tgctgcatga agagacccct 780

ccaaccacaa gttcagattc cgaagaggaa caggaggacg aggaagagat cgatgtggtc 840

tctgtggaaa agcgccaggc tccaggaaaa cgaagcgagt ccggctctcc aagtgcagga 900

ggacactcca agccacctca ttctcccctg gtgctgaaaa ggtgccacgt ctccacccac 960

cagcataact acgcagcccc accctctaca cgaaaggact atccagctgc aaagcgcgtg 1020

aaactggata gcgtgagagt cctgaggcag atcagtaaca atcggaagtg tacttcaccc 1080

agaagctccg acaccgaaga gaacgtgaaa aggcgcaccc ataatgtcct ggaacgccag 1140

cgacggaatg agctgaagag gtccttcttt gccctgcgcg atcagattcc tgaactggag 1200

aacaatgaga aggctccaaa agtggtcatt ctgaagaaag ccacagctta catcctgtct 1260

gtgcaggccg aagagcagaa actgatcagt gaagaggacc tgctgagaaa acgcagggaa 1320

cagctgaaac ataaactgga acagctgaga aactcttgtg cttaa 1365

<210> 8
<211> 3399
<212> DNA
<213> Artificial Sequence

<220>
<223> nucleotide sequence of hTERT

<400> 8
atgcccagag ctcccagatg cagagccgtg agaagcctgc tgagaagcca ctacagagag 60

gtgctgcccc tggccacctt cgtgagaaga ctgggacccc agggctggag actggtgcag 120

agaggcgacc ccgcagcctt tagagccctg gtggcccagt gcctggtgtg cgtgccctgg 180

gacgccagac ctcctcccgc tgcccccagc ttcagacagg tgagctgcct gaaggagctg 240

gtggccagag tgctccagag actgtgcgag agaggcgcca agaacgtgct ggcctttggc 300

ttcgccctgc tggatggagc cagaggcgga cctcccgagg ccttcaccac aagcgtgaga 360

agctacctgc ccaacaccgt gaccgatgcc ctgagaggct ccggcgcctg ggggctgctc 420

ctgagaagag tgggcgacga cgtgctggtg cacctgctgg ccagatgcgc cctgttcgtg 480

ctggtggctc ccagctgcgc ctaccaggtg tgcggacccc ctctgtacca gctgggagcc 540

gccacccagg caagaccccc tccccacgcc tctggaccca gaagaagact gggctgcgag 600

agagcctgga accacagcgt gagagaggct ggcgtgcccc tgggcctgcc cgcccctggc 660

gccagaagaa gaggcggcag cgccagcaga agcctgcccc tgcccaagag acccagacgc 720

ggcgccgctc ccgagcctga gagaacaccc gtgggccagg gcagctgggc ccaccccggc 780

agaaccagag gacccagcga cagaggcttc tgcgtggtga gccctgccag acccgccgag 840

gaggccacca gcctggaggg cgccctgagc ggcaccagac acagccaccc cagcgtgggc 900

agacagcacc acgccggccc tcctagcacc agcagacccc ccagaccttg ggacaccccc 960

tgcccccctg tgtacgccga gaccaagcac ttcctgtaca gcagcggcga caaggagcag 1020

ctgagaccca gcttcctgct gagctccctg agacccagcc tgaccggcgc cagaagactg 1080

gtggagacca tcttcctggg cagcagaccc tggatgcccg gcacccccag aagactgccc 1140

agactgcccc agagatactg gcagatgaga cccctgttcc tggagctgct gggcaaccac 1200

gcccagtgcc cctacggcgt gctgctgaag acccactgcc ccctgagagc tgccgtgacc 1260

cccgcagctg gcgtgtgcgc cagagagaag ccccagggca gcgtggccgc tcccgaggag 1320

gaggacaccg atcccagaag actggtgcag ctgctgagac agcacagcag cccctggcag 1380

gtgtacggct tcgtgagagc ctgcctgaga agactggtgc ctcccggcct gtggggcagc 1440

agacacaacg agagaagatt cctgagaaac accaagaagt tcatcagcct gggcaagcac 1500

gccaagctga gcctccagga gctgacatgg aagatgagcg tgagagactg cgcctggctg 1560

aggagaagcc ctggcgtggg ctgcgtgccc gccgccgagc acagactgag agaggagatc 1620

ctggccaagt ttctgcactg gctgatgagc gtgtacgtgg tggagctgct gagaagcttc 1680

ttctacgtga ccgagaccac attccagaag aacagactgt tcttttacag gaagagcgtg 1740

tggagcaagc tccagagcat cggcatcaga cagcacctga agagagtgca gctgagagag 1800

ctgagcgagg ccgaggtgag acagcacaga gaggccagac ccgccctgct gaccagcaga 1860

ctgagattca tccccaagcc cgatggcctg agacccatcg tgaacatgga ctacgtggtg 1920

ggagccagaa cctttagaag agagaagaga gccgagagac tgaccagcag agtgaaggcc 1980

ctgttcagcg tgctgaacta cgagagagcc agaagacccg gcctgctggg cgccagcgtg 2040

ctgggcctgg acgacatcca cagagcctgg agaaccttcg tgctgagagt gagagcccag 2100

gaccctcctc ccgagctgta cttcgtgaag gtggacgtga ccggcgccta cgacaccatc 2160

ccccaggaca gactgaccga ggtgatcgcc agcatcatca agccccagaa cacctactgc 2220

gtgagaagat acgccgtggt gcagaaggcc gcccacggcc acgtgagaaa ggccttcaag 2280

agccacgtga gcaccctgac cgacctccag ccctacatga gacagttcgt ggcccacctc 2340

caggagacca gccccctgag agatgccgtg gtgatcgagc agagctcttc cctgaacgag 2400

gcctccagcg gcctgttcga cgtgttcctg agattcatgt gccaccacgc cgtgagaatc 2460

agaggcaaga gctacgtgca gtgccagggc atcccccagg gcagcatcct gagcaccctg 2520

ctgtgcagcc tgtgctacgg cgacatggag aacaagctgt tcgctggcat cagaagagac 2580

ggcctgctgc tgagactggt ggacgacttc ctgctggtga ccccccacct gacccacgcc 2640

aagaccttcc tgagaaccct ggtgagaggc gtgcccgagt acggctgcgt ggtgaacctg 2700

agaaagaccg tggtgaactt tcccgtggag gacgaggccc tgggcggcac cgccttcgtg 2760

cagatgcccg cccacggcct gtttccctgg tgcggcctgc tcctcgacac cagaaccctg 2820

gaggtgcaga gcgactacag cagctacgca agaaccagca tcagagccag cctgaccttc 2880

aacagaggct tcaaggccgg cagaaacatg agaagaaagc tgttcggcgt gctgagactg 2940

aagtgccaca gcctgttcct ggacctccag gtgaacagcc tccagaccgt gtgcaccaac 3000

atctacaaga tcctgctgct ccaggcctac agattccacg cctgcgtgct ccagctgccc 3060

ttccaccagc aggtgtggaa gaatcccacc ttcttcctga gagtgatcag cgacaccgcc 3120

agcctgtgct acagcatcct gaaggccaag aatgccggca tgagcctggg cgccaagggc 3180

gccgctggac ccctgcccag cgaggccgtg cagtggctgt gccaccaggc cttcctgctg 3240

aagctgacca gacacagagt gacctacgtg cccctgctgg gcagcctgag aaccgcccag 3300

acccagctga gcagaaagct gcctggcaca accctgaccg ccctggaggc agccgcaaac 3360

cccgccctgc ccagcgactt caagaccatc ctggactag 3399

<210> 9
<211> 747
<212> DNA
<213> Artificial Sequence

<220>
<223> nucleotide sequence of tTA(tetracycline transactivator)

<400> 9
atgtcaaggc tggataaaag caaagtgatt aactccgctc tggaactgct gaacgaagtc 60

ggcattgagg ggctgaccac acgcaagctg gcacagaagc tgggagtgga gcagcccacc 120

ctgtactggc acgtgaagaa caagcgcgcc ctgctggacg ccctggccat cgagatgctg 180

gatcggcacc acacacactt ctgccctctg gagggcgaga gctggcagga cttcctgcgg 240

aacaatgcca agagctttag atgtgccctg ctgtcccaca gggatggagc aaaggtgcac 300

ctgggcacca gaccaacaga gaagcagtac gagaccctgg agaaccagct ggccttcctg 360

tgccagcagg gcttttctct ggagaatgcc ctgtatgccc tgagcgccgt gggacacttc 420

accctgggat gcgtgctgga ggaccaggag caccaggtgg ccaaggagga gagagagaca 480

cctaccacag actccatgcc ccctctgctg aggcaggcca tcgagctgtt tgatcaccag 540

ggcgccgagc cagccttcct gtttggcctg gagctgatca tctgcggcct ggagaagcag 600

ctgaagtgtg agtctggagg accagcagat gccctggacg atttcgacct ggatatgctg 660

cccgccgacg ccctggacga ttttgatctg gacatgctgc ctgctgatgc cctggatgat 720

tttgacctgg atatgctgcc tggataa 747

<210> 10

<211>    20
<212>    DNA
<213>    Artificial Sequence

<220>
<223>    Forward primer for BDNF


<400>    10
gcatcgacaa gagacactgg                                                    20


<210>    11
<211>    20
<212>    DNA
<213>    Artificial Sequence

<220>
<223>    Reverse primer for BDNF


<400>    11
caacaccacg gaattgtcag                                                    20


<210>    12
<211>    375
<212>    DNA
<213>    Artificial Sequence

<220>
<223>    nucleotide sequence of ZeoR


<400>    12
atggccaagt tgaccagtgc cgttccggtg ctcaccgcgc gcgatgtggc cggagcggtc        60

gagttctgga ccgaccggct cgggttcagc cgggacttcg tggaggacga cttcgccggt       120

gtggtccggg acgacgtgac cctgttcatc agcgcggtcc aggaccaggt ggtgccggac       180

aacaccctgg cctgggtgtg ggtgcgcggc ctggacgagc tgtacgccga gtggtcggag       240

gtcgtgtcca cgaacttccg ggacgcctcc gggccggcca tgaccgagat cggcgagcag       300

ccgtgggggc gggagttcgc cctgcgcgac ccggccggca actgcgtgca cttcgtggcc       360

gaggagcagg actaa                                                        375


<210>    13
<211>    600
<212>    DNA
<213>    Artificial Sequence

<220>
<223>    nucleotide sequence of PuroR


<400>    13
atgaccgagt acaagcccac ggtgcgcctc gccacccgcg acgacgtccc cagggccgta        60

cgcaccctcg ccgccgcgtt cgccgactac cccgccacgc gccacaccgt cgatccggac       120

cgccacatcg agcgggtcac cgagctgcaa gaactcttcc tcacgcgcgt cgggctcgac 180

atcggcaagg tgtgggtcgc ggacgacggc gccgcggtgg cggtctggac cacgccggag 240

agcgtcgaag cgggggcggt gttcgccgag atcggcccgc gcatggccga gttgagcggt 300

tcccggctgg ccgcgcagca acagatggaa ggcctcctgg cgccgcaccg gcccaaggag 360

cccgcgtggt tcctggccac cgtcggcgtc tcgcccgacc accagggcaa gggtctgggc 420

agcgccgtcg tgctccccgg agtggaggcg gccgagcgcg ccggggtgcc cgccttcctg 480

gagacctccg cgccccgcaa cctccccttc tacgagcggc tcggcttcac cgtcaccgcc 540

gacgtcgagg tgcccgaagg accgcgcacc tggtgcatga cccgcaagcc cggtgcctga 600

600

## Claims

1. A method for preparation of an immortalized stem cell line, comprising:
preparing a stem cell line by introducing an immortalizing gene;
introducing a gene encoding a foreign protein into the stem cell line;
cryopreserving the stem cell line; and
irradiating radiation to the cryopreserved stem cell line.

2. The method of claim 1, wherein the immortalizing gene is c-Myc and/or hTERT.

3. The method of claim 1, wherein the foreign protein is selected from the group of growth factors, cytokines or cancer therapeutic proteins, antibodies, and chemokine receptors.

4. The method of claim 1, wherein the immortalized stem cell line is a human embryonic stem cell (hES), a bone marrow stem cell (BMSC), a mesenchymal stem cell (MSC), a human neural stem cell (hNSC), a limbal stem cell, or an oral mucosal epithelial cell.

5. The method of claim 1, wherein the radiation is irradiated so that the absorbed dose to the stem cell line is at least 80 Gy.

6. The method of claim 5, wherein the absorbed dose to the stem cell line satisfies, with respect to irradiation dose, the following range:
80 to 140% of the irradiation dose when the irradiation dose is 0 to 100 Gy;
80 to 140% of the irradiation dose when the irradiation dose is 100 to 200 Gy; and
60 to 140% of the irradiation dose when the irradiation dose is 200 Gy or more.

7. The method of claim 1, wherein the radiation is selected from the group of gamma rays, beta rays, neutron rays, X-rays and electron beams.

8. A pharmaceutical composition comprising the immortalized stem cell line according to the method of claim 1.

9. The pharmaceutical composition of claim 8, wherein the pharmaceutical composition comprises an immortalized stem cell line expressing brain-derived neurotrophic factor (BDNF).

10. The pharmaceutical composition of claim 9, wherein the pharmaceutical composition has a preventive or therapeutic effect on neurological diseases.

11. The pharmaceutical composition of claim 10, wherein the neurological disease is selected from the group consisting of Alzheimer's disease (AD), Parkinson's disease (PD), amyotrophic lateral sclerosis (ALS), cerebral infarction, chronic brain injury, spinal cord injury, Huntington's disease (HD), Rett's disease (RD), ischemic brain disease, stroke and traumatic brain injury, neonatal hypoxic ischemic encephalopathy, and multiple sclerosis.

12. Use of the immortalized stem cell line according to the method of claim 1 as a cell therapeutic agent.

13. A method for treating a disease by administering the immortalized stem cell line according to the method of claim 1 in a therapeutically effective amount to a subject in need thereof.
